(19) Europäisches Patentamt
European Patent Office
Office européen des brevets

(11) Publication number: **0 427 620 A1**

(12) **EUROPEAN PATENT APPLICATION**

(21) Application number: **90403149.9**

(51) Int. Cl.[5]: **B01D 53/14**, C01B 13/02

(22) Date of filing: **07.11.90**

(30) Priority: **09.11.89 FR 8914723**

(43) Date of publication of application:
**15.05.91 Bulletin 91/20**

(84) Designated Contracting States:
**AT BE CH DE DK ES FR GB GR IT LI LU NL SE**

(71) Applicant: **L'AIR LIQUIDE, SOCIETE ANONYME POUR L'ETUDE ET L'EXPLOITATION DES PROCEDES GEORGES CLAUDE**
**75, Quai d'Orsay**
**F-75007 Paris(FR)**

Applicant: **AQUANAUTICS CORPORATION (a Delaware corporation)**
**980 Atlantic Avenue, No. 101**
**Alameda, California 94501(US)**

(72) Inventor: **Boisselier-Cocolios, Brigitte**
**44, rue de Marcoussis**
**F-91470 Limours(FR)**
Inventor: **Lagrange, Gilles**
**18, rue de la Biche Frette**
**F-91470 Forges Les Bains(FR)**
Inventor: **Draskovic, François**
**6, rue Jean-Philippe Rameau**
**F-78330 Fontenay Le Fleury(FR)**
Inventor: **Cocolios, Panayotis**
**44, rue de Marcoussis**
**F-91470 Limours(FR)**

(74) Representative: **Le Moenner, Gabriel et al**
**L'AIR LIQUIDE, Société Anonyme pour l'étude et l'exploitation des procédés Georges Claude 75, Quai d'Orsay**
**F-75007 Paris(FR)**

(54) **Process for the separation of a gas from a gas mixture using metal complexes of cyclic polynitrogenated derivatives.**

(57) This invention relates to a process for the separation of a gas from a gas mixture by using polynitrogenated cyclic complexes of a metal in which the pH and potential conditions applied are especially advantageous to perform the separation with an optimum yield.

This invention also relates to the application of this process for the separation of a pure gas starting from a mixture of gases ,or for the purification of a gas or of a gas mixture.

EP 0 427 620 A1

# PROCESS FOR THE SEPARATION OF A GAS FROM A GAS MIXTURE USING METAL COMPLEXES OF CYCLIC POLYNITROGENATED DERIVATIVES

This invention relates to a process of separation of a gas from a gas mixture by selective absorption of a gas molecule by an appropriate metal complex of a cyclic polynitrogenated derivative, followed by the unloading of the absorbed gas molecule by electrochemical activation of the gas molecule carrier.

This invention also relates to the purification of a gas that does not interact with the above-mentioned metal complexes and that is polluted by other gases which react with metal complexes of cyclic polynitrogenated derivatives and get absorbed.

The cyclic polynitrogenated derivatives are able to complex several transition metals when these transition metals are reacted with the cyclic polynitrogenated derivatives as metal salts. The most common transition metals found in the literature associated to the above-mentioned derivatives are Mn, Fe, Co, Ni, Cu, Zn. This list is not exhaustive. Specific interactions are also known to occur between several gas molecules and transition metal complexes. As examples one can mention the coordination of dioxygen, $O_2$, with iron, manganese, cobalt, rhodium, iridium or copper complexes, the coordination of carbon monoxide, CO, with copper, nickel, rhodium and iridium complexes, the coordination of carbon dioxide, $CO_2$, by cobalt, copper, nickel, zinc, ruthenium or platinum complexes, the coordination of nitrogen oxide, NO, by manganese, iron or cobalt complexes, the fixation of hydrogen, $H_2$, by palladium, platinum, ruthenium and molybdenum complexes, as described in the literature.

The organic derivatives that bind the transition metal, also designed as ligands, may be linear or cyclic, saturated or unsaturated, and possess one or several atoms of non metal groups, which are capable of chemical bondings with a metal. Such atoms can be found among the following, but are not limited to, nitrogen, phosphorus, oxygen or sulphur.

Among the organic ligands found in the literature, the following may be mentioned as efficient complexes of transition metals : the linear polyalkylamines, the phosphines, the phosphites, the phosphine oxides, the isocyanates, the Schiff bases, the porphyrins, the phtalocyanins, the cyclic polyamines.

In this invention, "complex" shall mean a complex of a transition metal with a ligand.

The coordination (or absorption) of a gas molecule by a transition metal complex may be used for the separation of a gas mixture. In order to produce a pure gas starting from a mixture of gases, the absorption step defined above must be followed by a decomplexation (or desorption) step and a complex treatment step, if needed, in order to get the complex back to its original dioxygen binding capacity.

The desorption step may be induced by different means such as the lowering of the partial pressure of the gas in the medium, with or without an increase of the temperature, or the chemical modification of the gas molecule carrier induced by a pH modification or by an oxydo-reduction reaction.

The chemical modification of the gas molecule carrier leads to electronic and/or structural changes of the metal environment such that they may induce the desorption of the absorbed gas molecule. As an example, it is posible to use a chemical oxydant that will interact either with the metal center or with the absorbed gas molecule and, doing so, induce the desorption of the absorbed molecule. The complex may then be regenerated, if needed, by use of a chemical reductant.

The same kind of modification may be performed with an appropriate electrochemical cell. The reduction will occur at the cathode and the oxidation at the anode. This invention is especially useful for the separation of dioxygen from a gas mixture, such as, for example, the air.

The separation of dioxygen from air has been described in several patents which differ from one another by the nature of the metal complex, the medium composition, the desorption mean and the complex regeneration.

As examples, the US patents # 4,451,270 and 4,542,010 claim cobalt complexes of linear or macrocyclic tetradentate ligands, of linear pentadentate ligands and of bidentate ligands of the Schiff base type, in the presence of a base for axial coordination, in non-aqueous media. The separation of dioxygen is effected by applying a pressure difference between the two faces of a gas permeable membrane.

The US patent # 4,442,297 and the european patent application # 0,220,963, claim complexes of manganese with phosphine ligands, used in anhydrous media, for the purification of nitrogen or the separation of dioxygen from air.

The US patent # 4,514,522 describes $O_2$ complexes with linear tetradentate ketoamine types of ligands, grafted on porous polymer. The US patent # 4,475,994 describes cobalt complexes in solvent mixtures at high pH that stabilize through a liquid membrane, the superoxide ion $O_2^{2-}$ generated by electroreduction.

More recently, the european patent application # 88 1027486 claims cobalt complexes of pentadentate polyalkylamine ligands in aqueous media, used for the separation of dioxygen from a fluid by mean of an

electrochemical process of desorption and regeneration of the complex.

However the process described in the above application, presents the following drawbacks :
- low yield in the gas absorption step ( about 50% )
- low yield in the gas desorption step.

The present invention provides a process for the separation of a gas from a gas mixture, which possess, compared to the known processes, the following advantages :
- the gas absorption kinetics on the complex, is greatly enhanced,
- the complex is used up to 100% of its absorption capacity
- up to 100% of the absorbed gas is recovered after the desorption step,
- the energy required for the production of a unit of gas is clearly reduced,
- the experimental conditions of this process indicate that a production unit will be an apparatus of very small dimensions.

This invention is especially illustrated by the following drawings :
- Figures 1 to 4 are titration curves of some cyclic polynitrogenated ligands carried out by $^1H$ NMR (Nuclear Magnetic Resonance) spectroscopy,
- Figure 5 is the titration curve by $^1H$ NMR spectroscopy, of the complex resulting from the reaction of a transition metal with a cyclic polynitrogenated ligand,
- Figure 6 is the standard curve for the titration, by $^1H$ NMR, of a complex in solution,
- Figure 7 compares the $^1H$ NMR titration curves of a cyclic polynitrogenated ligand and of its complex with a transition metal,
- Figure 8 shows the UV-Visible spectra of a complex under inert atmosphere and under dioxygen atmosphere,
- Figure 9 represents the variation of the dioxygen absorption by a complex as a function of the initial pH of the medium,
- Figure 10 is the voltammogram of a complex recorded under inert atmosphere,
- Figure 11 is the voltammogram of a complex recorded under air,
- Figure 12 shows the voltammograms of a complex, recorded under air at several pH values,
- Figure 13 is the voltammogram of a complex recorded in buffered medium under air,
- Figure 14 is the efficiency of the electrochemical dioxygen desorption plotted as a function of time.

This invention relates to a process for the separation of a gas from a gas mixture, this mixture being eventually dissolved in a liquid, characterized by the following steps:
- contacting the gas mixture containing the gas to be extracted with a solution in water or in a solvent mixture that dissolves $OH^-$ ions, or an organic base of a complex LM resulting from the coordination of a metal M, preferably chosen among the following metals: Ni, Co, Cu, Fe, Mn, Cr, V, Zn, and a ligand L of formula

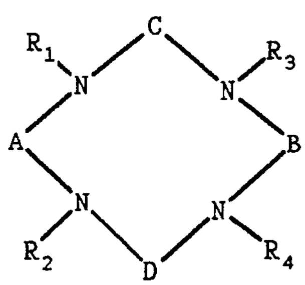

(I)

where,

. the A, B, C and D moieties, identical or different, represent :
- an alkyl chain $-(CH_2)_x-$, in which x represents an integer ranging from 2 to 4, substituted or not by one or several groups P or by one or several groups $P_1$, where $P_1$ is an alkyl group of 1 to 4 carbon atoms, substituted or not by one or several groups P, where P represents :
* a -COR group in which R represents a hydroxyle (-OH), a primary amine or an amine substituted by one or two alkyl groups of 1 to 4 carbon atoms, an $-OR'$ function in which $R'$ represents an alkyl group of 1 to 4 carbon atoms, or an aromatic cycle of 6 to 14 carbon atoms,
* an aromatic cycle of 6 to 14 carbon atoms, substituted or not in its ortho and/or meta and/or para position by a halide, an alkyl group of 1 to 4 carbon atoms, an alkoxy group of 1 to 4 carbon atoms, a hydroxy, an aryl group, an aromatic heterocycle, a nitro group, a group $-(CH_2)_u-COR$ in which R has the same meaning as above and u is an integer ranging from 0 to 4, or an amine, primary or substituted by 1 to 4 carbon

atoms,

* an aromatic heterocycle, especially containing a nitrogen atom, of 4 to 12 carbon atoms, substituted or not in its ortho and /or meta and/or para position by a halide, an alkyl group of 1 to 4 carbon atoms, an alkoxy of 1 to 4 carbon atoms, a hydroxy, an aromatic heterocycle, an aryle group, a nitro group, a group $-(CH_2)_u-COR$ in which R has the meaning indicated above and u represents an integer ranging from 0 to 4, or a primary amine or an amine substituted by one or two alkyl groups of 1 to 4 carbon atoms,
* a primary amine group or an amine substituted by one or two alkyl groups of 1 to 4 carbon atoms,
* a CN group,
* an alkyl group of 1 to 4 carbon atoms,
* an alkoxy group of 1 to 4 carbon atoms,
* a hydroxyl function,
* a nitro group,
* a halide,

- an aromatic cycle of 6 to 14 carbon atoms, in which two carbon atoms respectively participate to a bond with the nitrogen atoms located on each side of the A,B,C or D moieties of formula (I), this aromatic cycle being substituted or not by one or several groups P and/or $P_1$, where P and $P_1$ have the same meaning as above,
- a group $-CH_2-E-CH_2-$ where E represents an aromatic cycle of 6 to 14 carbon atoms, substituted or not by one or several groups P and/or $P_1$, where P and $P_1$ have the same meaning as above,

. $R_1$, $R_2$, $R_3$ and $R_4$, identical or different, represent a hydrogen atom or a group P or $P_1$, where P and $P_1$ have the same meaning as above,

this contacting being effected during a sufficient length of time for a complete formation of the complexes between the metal complexes LM as described above and the gas molecule to be extracted,

- transferring the obtained bulk in the anode compartment of an electrochemical cell, this cell containing electrodes, a cathode and an anode, respectively of high hydrogen overpotential and of high oxygen overpotential, preferably made of carbon,
- application, between the two cell compartments, of a potential high enough to induce the oxidation of the complexes formed with LM and the gas molecule, the unloading of the gas molecule in the vicinity of the anode and the reduction at the cathode of the complex LM after the above mentioned oxidation,
- recovery of the gas at the exit of the anode compartment, especially with a gas-liquid separation tower,
- if necessary, recovery and recycle of the LM complexes coming out of the cathode compartment into the operating cycle described above.

In a general manner, the ligand used in the process of this invention is chosen among the cyclic polyamines known, by a person skilled in the art, to complex a metal, and preferably among the ligands of formula (I) mentioned above.

- in a preferred embondiment of the invention the pH of the medium containing the complexes of LM with the gas molecule to be separated is adjusted at a value ranging from 7 to 14, and especially from 8 to 12 by addition of a strong inorganic base or by addition of an organic base as long as it does not permit the oxidation of the OH- ions or of the organic base in the next steps, by adding a strong inorganic base, such as but not limited to sodium or potassium hydroxide or by adding an organic base such as but not limited to 3,5-lutidine, 2,6-lutidine or triethylamine, then by adding an appropriate amount of a buffered solution in the case the added base is an inorganic base.

The pH adjusting step leading to a medium with a pH value ranging from 7 to 14 and, preferably from 8 to 12 but such that no oxidation of the $OH^-$ ions or of the added organic base occurs in the next step (which would induce a decrease of the pH and destroy the complex), as well as the addition step of a buffered solution in the case where an inorganic base is added, are both especially advantageous for the following step, the electrochemical desorption of the gas, to be effected with a yield of about 100%.

Modifying the pH as mentioned above, is advantageously realised by adding a strong inorganic base, such as but not limited to potassium or sodium hydroxide, or an organic nitrogeneous base such as but not limited to 3,5-lutidine, 2,6-lutidine, 2,4,6-collidine or triethylamine.

The above mentioned buffered solution , used during the different steps described above, is advantageously made of carbonate or hydrogenocarbonate ions, especially potassium carbonates or potassium hydrogenocarbonates, which can be mixed with sulfate ions, especially potassium sulfates.

When the pH of the solution is adjusted by addition of an organic base et more particularly by addition of an organic nitrogeneous base the system is self-buffered and no more extra buffer is needed. Several organic nitrogeneous bases are available which permit to reach pH values from 5 to 12 pH units. Among them one can mention pyridine and substituted pyridines, cyclic and linear amines substituted or unsubstituted.

In a preferred embodiment of this invention, the potential applied between the two cell compartments is ranging from 0.2 to 2V and more specifically from 0.5 to 1.8V, this potential being slightly higher than the sum of the redox potential value of the species oxidized at the anode, i.e. the complex formed with LM and the gas molecule, and of the redox potential value of the species reduced at the cathode, i.e. the LM complex resulting from the electrochemical desorption. The redox potentials mentioned above can be found in the literature or easily determined by a person skilled in the art, by using classical electrochemical analytical techniques.

This invention relates especially to a process which presents, before the realisation of the steps described above, the following steps :

- dissolving ligand L in water or in a solvent mixture where $OH^-$ ions are soluble,
- adding a strong base, especially sodium or potassium hydroxide, or an organic non-coordinating base such as but not limited to 2,6-lutidine or 2,4,6-collidine in such a quantity that the pH of the above mentioned solution becomes higher than the value of the $pK_{An}$, i.e. the ($-\log K_{An}$) value where $K_{An}$ represents the last protonation equilibrium constant of ligand L, which is expressed as the ratio of the product of the L and $H^+$ concentrations over the concentration of the monoprotonated ligand $LH^+$, in this solution,
- adding a known amount of a metal salt of formula $M_nX_m$, where M represents a transition metal at the oxidation state m+, and X represents an anion of charge n-, preferably chosen among $Cl^-$,$ClO_4^-$, $NO_3^-$, $CO_3^{2-}$, $SO_4^{2-}$, this amount being greater or preferably equal to one equivalent per ligand equivalent, and leaving the reaction proceed during a sufficient length of time so that the metal complex, LM, is formed
- if necessary, adjusting the pH by adding a strong inorganic base, especially sodium or potassium hydroxide or an organic base, either coordinating or non-coordinating especially 3,5-lutidine, 2,6-lutidine, 2,4,6-collidine or triethylamine, in order to maintain the pH at the value obtained in the preceeding step.

The addition of a strong inorganic base or a strong non-coordinating organic base in the medium containing the ligand L, facilitates the metal complexation by this ligand, which is performed with a yield of 50 to 100% and preferably with a yield of 85 to 95%.

Such pH conditions are also preferred for the gas absorption step which is performed with a yield of 80 to 95%.

In a preferred embodiment of this invention, the above described process is used for the production of a given gas, preferably pure, starting from a gas mixture. In that kind of process, the gas to be extracted from the gas mixture is the gas to be produced and then is collected at the exit of the anode compartment, especially with separation tower as described above.

Another preferred embodiment of this invention relates to the above described process where the electrochemical steps are performed under high pressure ranging from 1 to 200 bar and preferably from 2 to 20 bar, and the produced gas is collected and stored under pressure.

This invention relates more specifically to a process as described above such that :

- the gas extracted is the dioxygen present in the air and the metal M complexed to the ligand L, is the cobalt, or
- the gas extracted is the carbon dioxide present in a mixture of gases and the metal M complexed by the ligand L, is chosen among metals such as : Co, Cu, Ni, Zn, Ru, Pt and preferably among metals such as Cu and Zn, or
- the gas extracted is the carbon monoxide present in a mixture of gases and the metal M complexed by the ligand L, is chosen among metals such as Cu, Ni, Rh, Ir, and preferably M is Cu, or
- the gas extracted is the nitrogen monoxide present in a mixture of gases and the metal M complexed to the ligand L, is chosen among metals such as Mn, Fe, Co and preferably M is Co, or
- the gas extracted is the hydrogen present in a mixture of gases and the metal M complexed to a ligand L is chosen among metals such as Pd, Pt, Ru, Mo and is preferably Mo.

This invention also relates to a gas purification or a gas mixture purification process, these gases being unreactive with the complexes defined above.

A special embodiment of this invention pertains to a process as described above, where the gas to be extracted from the gas mixture, is an impurity that one wishes to eliminate, this process being then applied to the purification of a gas or a gas mixture, where the extracted gas is eliminated at the exit of the anode compartment while is collected the gas or gas mixture at the exit of the absorption tower situated right before the entrance of the anode compartment.

As mentioned previously, this process will be advantageously performed under pressure ranging from 1 to 200 bar and preferably from 2 to 20 bar, so that the gas produced is directly collected and stored at the desired pressure.

This invention will be especially illustrated by the following description of the experimental conditions used for the extraction of dioxygen from air, but is not limited to.

a) **Study and Optimisation of the dioxygen absorption**

The complexation of several metal ions by macrocyclic polynitrogenated ligands as well as their reactivity towards dioxygen are described in the literature. As examples, ENDICOTT et al. in Inorg. Chem., 28, 4011-4018 (1989), J. Amer. Chem. Soc., 111, 7411-7420 (1989), KIMURA et al. in Inorg. Chem., 25, 3883-3886 (1986), J. Coord. Chem., 15, 1-28 (1986), HANCOCK et al., Inorg. Chem., 25, 2160-2163 (1986), FABRIZZI et al., Inorg. Chem., 28, 3362-3366 (1989), PAOLETTI et al., Inorg. Chem., 28, 2480-2482 (1989) and references cited therein, describe kinetic studies of the complexation of metal ions, kinetic studies of dioxygen absorption, studies of the effect of the size of the polynitrogenated macrocycle and of the effect of the axial ligand on the above mentioned kinetics and on the lifetime of the oxygenated and deoxygenated complexes.

Generally, the above kinetic studies indiquate that the metal ion complexation is very slow. As an example, according to KIMURA et al. in Inorg. Chem., 25, 3883-3886 (1986), the complexation of $Ni^{2+}$ by the cyclam has reached its equilibrium, at pH 7, after one week. HANCOCK et al. in Inorg. Chem., 23, 1487-1489 (1984) explains that the presence of protonated cyclic polynitrogenated ligands in the solution decreases the complexation rate of the metal ions by electrostatic repulsions between the $-NH_2^+$ and $M^{n+}$ moieties and suggests the use of N-substituted derivatives with non-or weakly coordinating substitutants in order to improve that kinetics. However it is noticeable that most of the authors have carried out their experiments at pH values close to neutral.

One of the preferred embodiment of this invention is the use of a ligand of formula (I) in the above described process of separation of dioxygen from a gas mixture in basic to highly basic conditions of pH and, more specifically in conditions of pH where all the coordinative amines of ligand (I) are deprotonated.

As an illustration of these advantageous conditions, the equilibria that are involved in the complexation of $Co^{2+}$ and in the absorption of $O_2$ by a tetraazamacrocycle ligand of formula (I), without N-substituant, are described hereafter :

$$LH_4^{4+} \rightleftarrows LH_3^{3+} + H^+ \quad (1) , \quad K_{A1} = \frac{(LH_3^{3+})(H^+)}{(LH_4^{4+})}$$

$$LH_3^{3+} \rightleftarrows LH_2^{2+} + H^+ \quad (2) , \quad K_{A2} = \frac{(LH_2^{2+})(H^+)}{(LH_3^{3+})}$$

$$LH_2^{2+} \rightleftarrows LH^+ + H^+ \quad (3) , \quad K_{A3} = \frac{(LH^+)(H^+)}{(LH_2^{2+})}$$

$$LH^+ \rightleftarrows L + H^+ \quad (4) , \quad K_{A4} = \frac{(L)(H^+)}{(LH^+)}$$

$$L + Co^{2+} \rightleftarrows LCo^{2+} \quad (5) , \quad K_M = \frac{(LCo^{2+})}{(L)(Co^{2+})}$$

$$LCo^{2+} + O_2 \rightleftarrows LCoO_2^{2+} \quad (6) , \quad K_1 = \frac{(LCoO^{2+})}{(O_2)(LCo^{2+})}$$

$$LCoO_2^{2+} + LCo^{2+} \rightleftarrows LCoO_2CoL^{4+} \quad (7) , \quad K_2 = \frac{(LCoO_2CoL^{4+})}{(LCoO_2^{2+})(LCo^{2+})}$$

or : $$2LCo^{2+} + O_2 \rightleftarrows LCoO_2CoL^{4+} \quad (8)$$

with $$K_{O_2} = K_1K_2 = \frac{(LCoO_2CoL^{4+})}{(LCo^{2+})^2(O_2)}$$

To these equilibria should be added intermediate equilibria relating to partially protonated ligands $LH_3^{3+}$, $LH_2^{2+}$, and $LH^+$ that also complex $Co^{2+}$ according to the general equation :

$xLH_y^{y+} + CO^{2+} \rightleftarrows ((LH_y)_xCo)^{(xy+2)+}$ (9)

where x = 1 to 4 and y = 1 to 3.

According to this invention, the initial pH of the aqueous solution must be adjusted, before introduction of the metal salt, to a value higher than the value of the $pK_{A4}$ of the ligand so that all its coordinative amines will be deprotonated. The solution will contain then mainly the ligand in its L form (equation 4) and the complexation of $Co^{2+}$ (equation 5) will not be prevented by the electrostatic repulsion forces mentioned by HANCOCK et al.. In such conditions, the cobalt complexation , under argon atmosphere, followed by UV-Visible spectrophotometry, leads to the record of the $LCo^{2+}$ absorption band at its maximum after a few

minutes only.

However, the preparation of the solution at the right pH cannot be performed if the $pK_{Ai}$ values of the ligand L and, more precisely if the $pK_{A4}$ are unknown. In order to determine them, the classical titrimetric techniques are currently used. Proton Nuclear Magnetic Resonance spectroscopy, $^1H$ NMR, described by Mc. DONALD et al. in J. Amer. Chem. Soc., 85, 3736-3742 (1963) may also be used. According to this method, the chemical shift of one hydrogen atom of a ligand L is due to an average of the contribution of all species $LH_n^{n+}$ present in solution. Since the presence of these species $LH_n^{n+}$ is a function of the solution pH, the chemical shift of a hydrogen atom of ligand L will vary if the pH is modified. The $\Delta\delta_{Hi}$ = f(pH) curves permit the evaluation of the $pK_{Ai}$ values or at least of the $pK_{A4}$ value. As examples, some experimental results are reproduced on Figures 1 to 4. The $pK_{Ai}$ values of several ligands of formula (I) measured from these curves, are summarized on Tables IA and IB. The person skilled in the art will then know the initial pH of the solution for a fast complexation of $Co^{2+}$.

The study of the cobalt complexation is realised by classical potentiometry. However, an excellent evaluation is obtained by use of $^1H$ NMR according to the experimental procedure described by Mc. DONALD et al., J. Amer. Chem. Soc., 85, 3736-3742 (1963). Experimental results from this work on the ligand 1,4,8,11-tetraazacyclotetradecane, or cyclam (ligand 1 of Table IA), are reported on Figure 5. The Figure 6 reproduces the standard curve necessary for the realisation of $\Delta\delta_{HOD}$ = f(pH), where HOD is the monodeuteriated water, also present in the solvent, $D_2O$, used for recording of the $^1H$ NMR spectra.

In the case of the cyclam, Figure 7, if one superimposes the $\Delta\delta_{Hi}$ = f(pH) and $\Delta\delta_{HOD}$ = f(pH) curves, respectively indicative of $pK_{A4}$ and $pK_M$ values, the pH window in which the cobalt ion is totally complexed by L is perfectly defined. The major species in solution is then the complex (hydroxo)(cyclam)Co(II), LCO-(OH)$^+$, also reported by CABANI et al., Inorg. Chim. Acta, 89, L21-L22 (1984).

**Table IA : Estimation of $pK_{Ai}$ by $^1H$ NMR and determination of the initial pH of the solution before addition of the Co(2+) salt.**

| Ligands | $pK_{Ai}$ | pH before addition of Co(2+) |
|---|---|---|
| 1 | 11.2 | 12.0 |
| 2 | 10.5  11.5 | 12.5 |
| 3 | 3.8  9.6  10.6  11.2 | 12.0 |
| 4 | 9.5  9.8  11.7 | 12.5 |
| 5 | 10.7 | 12.0 |
| 6 | 2.5  6.9  10.5 | 11.5 |
| 7 | ** | |
| 8 | ** | |

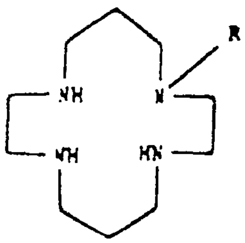

| R = | |
|---|---|
| H | 1 |
| $CH_2CO_2H$ | 2 |
| $CH_2CH_2CO_2H$ | 3 |
| $CH_2C_6H_5$ | 4 |
| $CH_2$ o-pyridine | 5 |
| $CH_2$ m-pyridine | 6 |
| $CH_2CH_2$ o-pyridine | 7 |
| $CH_2CH_2CH_2NH_2$ | 8 |

Ligands 2 and 3 are used as their chlorhydrate salt.
** NMR titration, is not applicable

**Table IB : Estimation of $pK_{Ai}$ by $^1H$ NMR and determination of the initial pH of the solution before addition of the Co(2+) salt.**

| Ligand | $pK_{Ai}$ | pH before addition of Co(2+) |
|---|---|---|
| 1 | 11.2 | 12.0 |
| 9 | 3.5 5.0 11.5 | 12.5 |
| 10 | ** | |
| 11 | 6.0 10.5 | 12.0 |
| 12 | ** | |

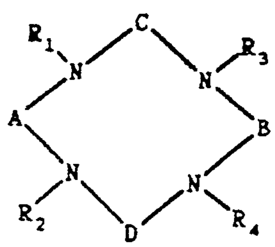

| A | B | C | D | |
|---|---|---|---|---|
| $(CH_2)_3$ | $(CH_2)_2$ | $(CH_2)_3$ | $(CH_2)_3$ | 1 |
| $(CH_2)_3$ | $(CH_2)_3$ | $(CH_2)_2$ | $(CH_2)_3$ | 9 |
| $(CH_2)_2$ | $(CH_2)_2$ | $(CH_2)_2$ | $(CH_2)_2$ | 10 |
| $(CH_2)_3$ | $(CH_2)_3$ | $(CH_2)_3$ | $(CH_2)_3$ | 11 |
| $(CH_2)_2$ | $(CH_2)_2$ | $(CH_2)_3$ | $(CH_2)_2$ | 12 |

** NMR titration, not applicable.

The introduction of dioxygen, in its pure form or as a mixture like air, in the solution prepared according to the procedure described above, i.e; : (i) dissolve ligand L in water, (ii) adjust the pH at a value higher than $pK_{A4}$, (iii) introduce one equivalent of a metal salt, (iv) adjust the pH at the previous value, if necessary, leads to the corresponding dioxygenated complex, according to the following reaction :

$2\ LCo(OH)^{+} + O_2 \rightleftharpoons (LCO(OH))_2O_2{}^{2+}$ (10)

without large modification of the bulk pH. This absorption reaction is also followed by UV-Visible spectrophotometry. The absorption band, characteristic of the oxygenated species at $\lambda$ = 285 nm is rather ill-defined and appears at higher wavelengths than the non-oxygenated complex. As an example the spectrum recorded for the $LCo(OH)^{+}$ complex, where L is the cyclam, is reproduced on Figure 8, under argon atmosphere (spectrum 8a) and under $O_2$ atmosphere (spectrum 8b).

It is also possible to determine the amount of dioxygen absorbed by volumetric measurements. The results obtained for several ligands L are summarized on Table II. When the initial pH is higher than 11.5, only a weak variation of the pH is observed during the formation of the oxygenated complex and the absorption is at its maximum since the ratio Number of moles of dioxygen absorbed / Number of moles of ligand L, $MO_2abs$ / ML, is close to 0.5, which is the value for a stoechiometric reaction leading to the formation of a 2:1 dioxygenated complex (equation 10). As an illustration, is reproduced on Figure 9, the ratio $MO_2abs$ / ML as a function of pH for the cyclam. The variation recorded confirms the above described observations. At pH values lower than 9, the $O_2$ absorption is very weak and is almost unnoticeable at

Table II

| Volumetric oxygen absorption measurements of $LCo^{2+}$ complexes. | | | | | |
|---|---|---|---|---|---|
| Ligand | ligand solution pH | pH Adjustment | Metal salt | pH after oxygenation | $MO_2$abs/ML |
| 1 | 11.73 | 7.02 | $Co(AcO)_2$ | 5.19 | 0 |
| | 11.14 | 9.00 | - | 5.12 | 0.025 |
| | 11.30 | 10.47 | - | 5.76 | 0.226 |
| | 11.19 | 10.53 | - | 5.71 | 0.297 |
| | 11.14 | 11.00 | - | 6.24 | 0.398 |
| | 11.78 | 11.13 | - | 6.58 | 0.450 |
| | 11.66 | 12.00 | - | 8.10 | 0.458 |
| | 11.75 | 12.00 | $CoCl_2$ | 11.75 | 0.464 |
| | 11.62 | 12.00 | $Co(NO_3)_2$ | 11.82 | 0.446 |
| | 11.80 | 12.00 | $CoSO_4$ | 8.25 | 0.453 |
| 2 | 1.40 | 8.00 | $Co(AcO)_2$ | 4.99 | 0.223 |
| | 1.50 | 8.53 | - | 4.95 | 0.236 |
| | 1.43 | 9.10 | - | 5.00 | 0.250 |
| | 1.36 | 12.02 | - | 11.92 | 0.465 |
| 3 | 1.76 | 8.00 | - | 5.14 | 0.162 |
| | 1.70 | 9.00 | - | 5.13 | 0.209 |
| | 1.46 | 12.01 | - | 11.93 | 0.456 |
| 4 | 11.01 | 11.01 | - | 6.82 | 0.154 |
| | 11.40 | 12.45 | - | 12.01 | 0.462 |
| 5 | 1.70 | 12.00 | $CoSO_4$ | 8.22 | 0.436 |
| 6 | 2.35 | 12.00 | $CoSO_4$ | 11.24 | 0.490 |
| 7 | 2.29 | 12.00 | $CoSO_4$ | 10.89 | 0.565 |
| 8 | * | * | $CoSO_4$ | * | 0.420 |
| 9 | 11.24 | 12.00 | $CoSO_4$ | 9.47 | 0.371 |
| 10 | 11.30 | 12.00 | $CoSO_4$ | 11.70 | 0.430 |
| 11 | 10.76 | 12.00 | $CoSO_4$ | 11.89 | 0.240 |
| 12 | 2.47 | 12.00 | $CoSO_4$ | 11.16 | 0.475 |

* not available

pH lower than 8. For pH values in the range 9 to 11.5, a large variation of the amount of $O_2$ absorbed is observed, which reaches its maximum at pH value higher than 12.

**b) Study of the electrochemical desorption of dioxygen.**

Only a few electrochemical studies on metal complexes of ligands of formula I and their dioxygenated complexes, have been reported in the literature. ANSON et al., J. Amer. Chem. Soc., 103, 7489-7496 (1981), describe the reduction of dioxygen into $H_2O_2$ electrocatalyzed on a graphite electrode in the presence of the complex $LCo(III)(OH_2)_2{}^{3+}$ where L represents the cyclam, at neutral pH. In the same reference, ANSON et al. also report the cyclic voltammetry of the corresponding dioxygenated complex $(LCo(OH_2))_2O_2{}^{4+}$. However, only the description of the reduction waves are detailed while the oxidation wave at 1.1V vs the Saturated Calomel Electrode (S.C.E.) is only mentioned without interpretation.

No electrochemical study has ever mentioned the desorption of dioxygen by electrochemical oxidation of the dioxygenated complexes of this invention.

The electrochemical behavior of the cobalt complexes prepared according to the above described procedure, have been studied by the authors by cyclic voltammetry, polarography on rotating disk electrodes, bulk electrolysis at controlled potential on carbon electrodes, in solution containing appropriate supporting electrolytes such as and without limitation, $ZClO_4$, $ZCl$, $ZNO_3$, $ZHCO_3$, $Z_2CO_3$, $Z_2SO_4$, $Z_3PO_4$ where Z is an alcalin, preferably Na and K. The potentials are measured versus the Saturated Calomel Electrode (S.C.E).

The complexes $LCo(OH)^+$ prepared at pH > $pK_{A4}$, under argon atmosphere, do not exhibit a reduction wave in the potential window of the solvent/supporting electrolyte. They are however oxidized at potentials close to 0.0V and the generated species are then reduced at potentials ranging from -0.5V to -1.5V and more precisely from -0.7 to -1.3V, the values being varied with the structure of ligand L, with the medium composition (pH, nature and concentration of the supporting electrolyte) and with the nature and the initial treatment of the electrode.

The corresponding dioxygenated complexes $(LCo(OH))_2O_2{}^{2+}$ are reduced, irreversibly, between 0.0V and -1.5V and more often between -0.5V and -1.2V, the values being varied with the structure of ligand L, with the medium composition (pH, nature and concentration of the supporting electrolyte) and with the nature and the initial treatment of the electrode.

These complexes $(LCo(OH))_2O_2{}^{2+}$ are irreversibly oxidized between 0.0V and +1.5V and more often between +0.3V and +0.8V, the values being varied with the above mentioned parameters, and present deep well defined waves, indicative of a fast electron transfer from the complex to the electrode. By reversing the potential scan, two reduction reactions are observed, the first one at potentials around -0.4V to -0.5V, attributed to the reduction of free dioxygen in solution, the second one, between -0.7V and -1.3V, attributed to the metallic species generated at the electrode during the oxidation step. For this last reduction, the parameters previously listed for other electrochemical reactions, will also control the potential value.

The reduction between -0.4V and -0.5V has been attributed to the reduction of dioxygen based on experiments carried out on (i) solutions containing different known quantities of dissolved $O_2$, without $LCo(OH)^+$ or $(LCo(OH))_2O_2{}^{2+}$ complexes, (ii) solutions of $(LCo(OH))_2O_2{}^{2+}$ that do not contain no free dioxygen.

The second reduction between -0.5V and -1.5V corresponds to the reduction observed for $LCo(OH)^+$ complexes in solution under argon atmosphere.

These cyclic voltammetry results are illustrated on Figures 10 and 11 for $LCo(OH)^+$ under argon atmosphere (Figure 10) and under air (Figure 11) and can be summarized by the following reaction schemes, ground of a continuous process of separation of dioxygen from a gas mixture.

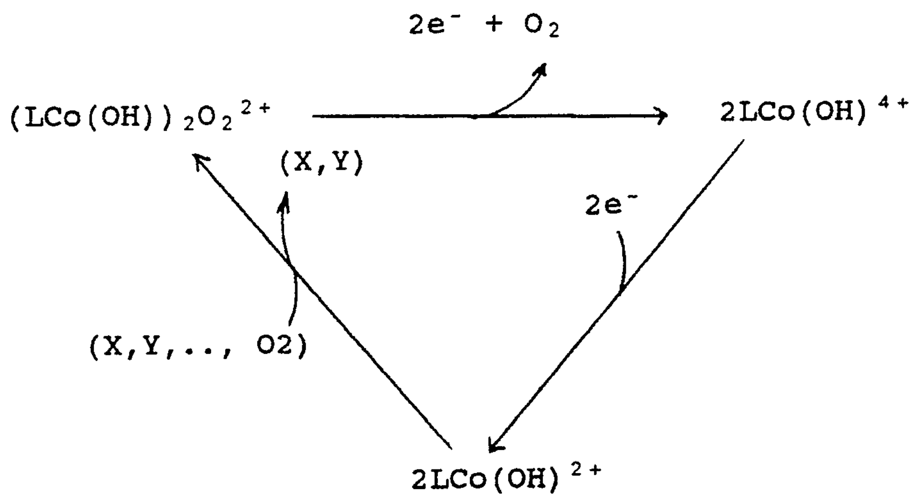


SCHEME I

or;

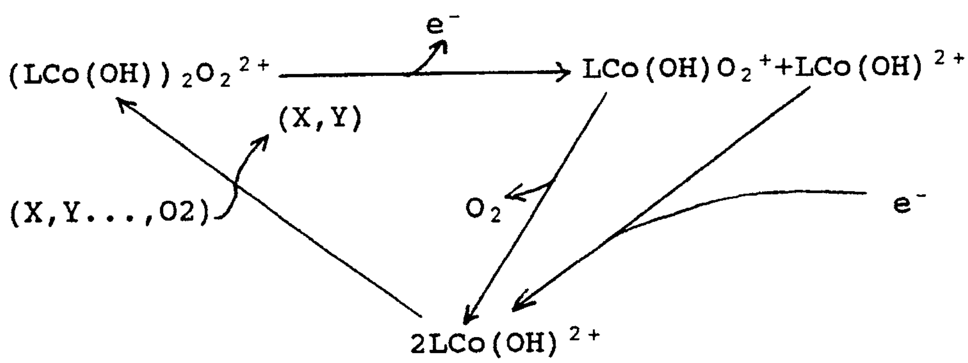


SCHEME II

A specific embodiment of this invention is the determination of the optimum pH for the best electron transfer kinetics in the oxidation of the dioxygenated complex without oxidizing the $OH^-$ species or the added organic base. The oxidation of the hydroxyl ions or the oxidation of the added organic base is effected at potentials more positive than the oxidation potentials of the dioxygenated species and is dependant on $OH^-$ concentration and thus on the pH. The study of the oxidation of $(LCo(OH))_2O_2^{2+}$ at different pH leads to the determination of the pH domain where the oxidation waves of $(LCo(OH))_2O_2^{2+}$ and $OH^-$ are distant enough while still keeping high current intensity for a significant production of $O_2$ per time unit and per electrode area unit.

As an illustration, such measurements are reproduced on Figure 12 where a person skilled in the art can easily observe that the optimum pH for the desorption of dioxygen by electrooxidation of the $(LCo(OH)-)_2O_2^{2+}$ complex ranges from 10 to 12 and more precisely is of 11.5 (Figure 12c).

Another specific embodiment of this invention is the definition of the choice of the appropriate supporting electrolyte for the process. A large variety of salts are indeed available, the cation being either from the first or second group of the periodic table. However the anion will play an important role in the process since it will buffer the solution in the optimum pH range defined previously and also it will participate to the coordination of the species generated by electrooxidation. These species are complexes of cobalt at the oxidation state III. The reduction potentials of such species depend on the axial ligand structure, which is usually for Co(III) complexes in ionic solutions, an anion. If coordinative anions are present at high concentration, ligands may be displaced according to the following equation :

$LCo(III)(OH)^{2+} + X^- \rightleftharpoons LCo(III)X^{2+} + HO^-$ (12)

This reaction may have a large influence on the economy of the process if the new species $LCo(III)X^{2+}$ is reduced at a lower potential than its precursor $LCo(III)(OH)^{2+}$, since the voltage applied between the anode and the cathode of an electrochemical cell is given by the expression :

$\Delta Emin = |Eox| + |Ered|$
where Eox and Ered are the potential values respectively of the oxidation and reduction steps.

In this invention, the buffer effect and the axial ligand effect have been combined by using as supporting electrolyte, a mixture of potassium sulfate, $K_2SO_4$, and potassium carbonate, $K_2CO_3$, in the ratio 1/10 and preferably in the ratio 1/1. The results obtained are reproduced on Figure 13 for the cyclam. After introduction of dioxygen, the bulk pH is 11.9, the oxidation potential of the oxygenated complex is 0.6V and the reduction potential of the species resulting from the oxidation step is -0.9V. The voltage to apply is then $\Delta Emin$ = 1.5V instead of 1.9V when the supporting electrolyte is $NaClO_4$.

Oxidation/reduction potentials obtained from Cyclic Voltammetry for selected oxygenated complexes are summarized on Table III.

Another imrpovement of the invention is the addition of organic bases and more specifically the addition of nitrogeneous organic bases which buffer the solution, i.e. they stabilize the pH of the solution. These nitrogeneous bases may be coordinating such as pyridine, 3,5-lutidine or triethylamine or non-coordinating such as 2,6-lutidine or 2,4,6-collidine (or mixtures of coordinating and non coordinating nitrogeneous bases) which cover a large pH range and more particularly from pH 5 to 12.

The coordinating nitrogeneous bases are especially use full for buffering solutions of tetracoordinating tetraazamacrocycle complexes such as complexes of ligands 1, 4, 9, 10, 11 and 12 (the appropriate structures are reported on Tables IA and IB).

Furthermore, at the same time these coordinating nitrogeneous bases contribute to the stabilization of the $LCo^{2+}$ and $(LCo)_2O_2{}^{2+}$ complexes of tetracoordinating tetraamacrocycles by acting as axial ligands. Another contribution of this axial coordination is the enhancement of the affinity of the cobalt complex to coordinate with a gas molecule and more specifically to coordinate with dioxygen, thus considerably improving both the absorption capacity and the absorption kinetics of the syntem. In order to optimize absorption capacity/kinetics and enhancement of the electrochemical desorption step one can advantageously use appropriate mixtures of coordinating and non-coordinating nitrogeneous organic bases depending on the specific physicochemical characteristics of the $LCo^{2+}$ derivative used, thus providing the system with maximum and fast loading of $O_2$, with fast unloading of $O_2$ and with enhanced life-time.

The defined operating conditions, when applied to controlled potential electrolysis experiments, lead to the desorption of dioxygen. Several experiments carried out with selected ligands and different supporting electrolytes, on felt carbon electrodes, at oxidation potentials ranging from +0.6 to 0.8V, have induced the desorption of $O_2$. Depending on the experimental conditions, the yields in desorbed dioxygen, measured with a volumeter, vary from 30 to 100% and the gas purity is greater than 98%. The current efficiency based on a one-electron oxidation mechanism (see scheme II) ranges from 15 to 60% and also depends on the experimental conditions used.

Experiments carried out without ligand and without cobalt, all other conditions being unchanged, have also been performed and no $O_2$ production has been observed while applying the same potential range. The solvent is affected ($OH^-$ oxidation), only at potential values greater than 1.6V.

## c) Examples of separation of dioxygen from air : total electrolysis at controlled potential

### 1) Equipment :

The experiments are carried out in a cell with two compartments separated by a fritted glass of medium porosity. The working electrode is of carbon felt (Carbone Lorraine, RVC 1000), the counter electrode is in platinum and the reference electrode is a saturated calomel electrode (S.C.E.). The potential is applied using an EG&G PAR 173 potentiostat coupled to an EG&G PARC 175 signal generator and the coulombs are recorded by an EG&G PARC 179 coulometer. The set-up also contains a tube for the introduction of gases into the solution, a pH electrode connected to a pH-meter, a magnetic stirrer, a water vapor condensation system maintained at 0° C and a dioxygen analyser (SERVOMEX OA 570) on line with a gas volumeter (BROOKS).

### 2) Experimental procedure :

The solubilization of the ligand, the complex formation, the dioxygen absorption and its electrochemical desorption are effected according to the following steps :

Step 1 : Supporting electrolyte solutions at a concentration of 0.5M (M = mole/liter) in water, are prepared and introduced in both compartments. More precisely 15 to 25 mL of the supporting electrolyte solution is introduced in the working compartment (anode compartment).

**TABLE III : Cyclic Voltametry Results**

| Ligand | mM | Supporting Electrolyte | pH | Form | $E_{initial}$ | Scan Direction | $E_{p,c}$,V | $E_{p,a}$,V |
|---|---|---|---|---|---|---|---|---|
| 1 | 100 | 1 | 11.51 | deoxy | -0.50 | + | -1.24 | +0.19 |
| 1 | 100 | 1 | 11.45 | oxy | -0.20 | + | -1.30 | +0.62 |
| 1 | 100 | 1 | 11.45 | oxy | -0.20 | - | -1.20 | +0.62 |
| 1 | 100 | 2 | 12.00 | deoxy | -0.10 | + | 0.93 | +0.63 |
| 1 | 100 | 2 | 11.82 | oxy | -0.10 | + | 1.18 | +0.64 |
| 1 | 100 | 2 | 11.82 | oxy | -0.10 | - | -1.29 | +0.64 |
| 1 | 100 | 3 | 11.75 | deoxy | -0.20 | + | ID | NON |
| 1 | 100 | 3 | 11.75 | oxy | -0.20 | + | NON | +0.71 |
| 1 | 100 | 4 | 12.21 | deoxy | -0.20 | + | -0.68 | -0.0 |
| 1 | 100 | 4 | 11.90 | oxy | -0.20 | + | -0.94 | +0.67 |
| 1 | 100 | 4 | 11.90 | oxy | -0.20 | - | -1.29 | +0.67 |
| 1 | 100 | 5 | 8.29 | deoxy | -0.50 | + | -0.67,-1.19 | +0.57 |
| 2 | 100 | 1 | 11.47 | deoxy | -0.60 | + | -0.71 | +0.21 |
| 2 | 100 | 1 | 11.19 | oxy | -0.20 | + | -0.75,-0.88 | +0.74 |
| 2 | 100 | 3 | 11.60 | deoxy | -0.50 | + | -0.86 | +0.60 |
| 2 | 100 | 3 | 11.58 | oxy | -0.30 | + | -0.92 | +0.54,CW |
| 2 | 100 | 3 | 11.58 | oxy | -0.30 | - | 0.89 | CW |
| 3 | 100 | 1 | 11.91 | deoxy | -0.20 | + | -0.78 | +0.57 |
| 3 | 100 | 1 | 10.96 | oxy | -0.20 | + | CW | +0.58,+0.81 |
| 3 | 100 | 1 | 10.96 | oxy | -0.20 | - | -0.72,CW | CW |

**TABLE III : Legend**

Ligand : (N-R)(1,4,8,11) tetraazacyclotetradecane

R = H **1** : R = $CH_2CO_2H$ **2**

R = $CH_2CH_2CO_2H$ **3**

Supporting Electrolyte : 1 = 0.5M $NaClO_4$

2 = 0.5M $K_2SO_4$

3 = 0.5M $K_2CO_3$

4 = 0.25M $K_2SO_4$ + 0.25M $K_2CO_3$

5 = 0.5M $KHCO_3$

Form : deoxy = deoxygenated complex

oxy = oxygenated complex

$E_{initial}$ : volts vs. SCE (Saturated Calomel Electrode)

Electrode : glassy carbon

NON = redox couple non observed

ID = ill defined

CW = complex waves

Step 2 : The ligand L is introduced in the anode compartment, then the pH is adjusted to an appropriate value, greater than the ligand $pK_{A4}$ value, by adding a volume of NaOH or KOH 2M solution.

Step 3 : The cobalt salt is introduced by small quantities at a time, under stirring, while keeping the pH at values greater than 11-11.5 by adding aliquots of 2M NaOH or KOH solution. The solution is kept under stirring until a brownish color is reached, characteristic of the $LCo(OH)^+$ complex, and no precipitate appears anymore in the solution.

Step 4 : Air is bubbled through the solution in order to form the dioxygenated species. During this step, the color changes from light brown to dark brown and the pH stays almost constant.

Step 5 : Pure dioxygen is introduced in the atmosphere above the bulk solution, in the analyzer and in the tubing to the volumeter.

Step 6 : The required potential is applied. Gas bubbles are readily observed on the electrode and then in the solution, as the volumeter indicates gas formation. The volume of dioxygen produced is thus recorded as a function of time.

Steps 1 to 3 may be performed under inert atmosphere or under air. If they are under air, the dark brown color indicating the formation of the dioxygen complex, is reached faster.

After complete electrolysis the solution is dark red, characteristic color of the $LCoIII(OH)^{2+}$ species. When this solution is reduced under inert atmosphere at an appropriate potential (see Table III), the light brown color typical of the $LCoII(OH)^{+}$ is recovered. When the pH is adjusted at values greater than 11-11.5, steps 4 to 6 are repeated several times with the same results. The experiments are carried out with 15 to 25 mL of aqueous solution containing a supporting electrolyte at the concentration of 0.5M or a mixture of supporting electrolytes at a total concentration of 0. 5M. The ligand and the cobalt salt are used in stoechiometric quantities at a concentration ranging from 0.06 to 0.1M.

The electrochemical performances are expressed by the faradic yield which is the current consumed by the reaction for the production of dioxygen, and the desorption efficiency, defined as the ratio of the number of moles of dioxygen produced over the theoretical number of moles of dioxygen absorbed. This last theoretical number is half of the number of moles of ligand (or of cobalt) used. On Table IV are summarized the results obtained and on Figure 14 is reproduced the desorption efficiency curve as a function of time, for the cyclam complexed to $CoClO_4$ in the presence of $NaClO_4$ as supporting electrolyte. In this last case, the desorption efficiency is 96.8% and the faradaic yield is 64%, assuming a one electron mechanism as described on Scheme II.

The study and optimization of the dioxygen absorption by a complex of formula I as well as the study of the electrochemical desorption of dioxygen are illustrated with the following figures :

- Figure 1 represents the titration curve of the cyclam, $\Delta\delta_{Hi}$ = f(pH) by $^1$H N.M.R.
- Figure 2 represents the titration curve of the N-acetyl cyclam, $\Delta\delta_{Hi}$ = f(pH) by $^1$H N.M.R.
- Figure 3 represents the titration curve of the N-propionyl cyclam, $\Delta\delta_{Hi}$ = f(pH) by $^1$H N.M.R.
- Figure 4 represents the titration curve of the N-benzyl cyclam, $\Delta\delta_{Hi}$ = f(pH) by $^1$H N.M.R.
- Figure 5 represents the titration curve

Table IV

| Controlled Potential Electrolysis. | | | | | | | | |
|---|---|---|---|---|---|---|---|---|
| L | Metal Salt | Supporting Electrolyte | Initial pH | Final pH | Applied Potential (V) | Desorbed Gaz Volume (Nml) | Desorbed Efficiency (%) | Faradic Yield % |
| 1 | $Co(ClO_4)$, $6H_2O$ | $NaClO_4$ | 11.32 | 7.51 | 0.70 | 17.2 | 59.4 | 39.0 |
| 1* | $Co(ClO_4)$, $6H_2O$ | $NaClO_4$ | 11.23 | 11.30 | 0.68 | 14.4 | 51.5 | 24.4 |
| 1 | $Co(ClO_4)_2$, $6H_2O$ | $NaClO_4$ | 11.30 | 8.11 | 0.80 | 28.2 | 96.8 | 64.0 |
| 1 | $CoSO_4$, $7H_2O$ | $K_2CO_3/K_2SO_4$ 3/2 | 11.94 | 11.34 | 0.70 | 10.4 | 35.9 | 16.8 |
| 1 | $CoSO_4$, $7H_2O$ | $K_2CO_3/K_2SO_4$ 3/2 | 11.95 | 10.55 | 0.80 | 11.8 | 40.0 | 17.6 |
| 2 | Co(ClO4)2, 6H2O | NaClO4 | 11.23 | 10.55 | 0.85 | 7.7 | 67.0 | 26.0 |
| 3 | Co(ClO4)2, 6H2O | NaClO4 | 11.00 | 10.55 | 0.85 | 2.1 | 11.0 | 12.5 |
| - | - | NaClO4 | 11.36 | 0.67 | 1.60 | 0.5 | - | - |

* +1.5 equivalents of $N(CH_2CH_3)_3$.

of the $Co^{2+}$(cyclam) complex, $\Delta\delta_{HOD}$ = f(pH) by $^1$H N.M.R.

- Figure 6 represents the standard curve of $Co^{2+}$ in solution, $\Delta\delta_{HOD}$ = f(($Co^{2+}$)), which is used for the titration of $LCo^{2+}$ complexes,
- Figure 7 is an $^1$H N.M.R. illustration of the $Co^{2+}$ complexation by the cyclam as a function of the initial pH of the ligand solution.
- Figure 8 reproduces the UV-Visible spectra of the complexes of $Co^{2+}$ with the cyclam, under argon, (a), and under dioxygen (b),
- Figure 9 shows the effect of the initial pH of the ligand solution on the dioxygen absorption, for complexes of cyclam with $Co^{2+}$,
- Figure 10 reproduces the voltammogram recorded under argon, for the $Co^{2+}$ complex of the cyclam, in the presence of sodium perchlorate as supporting electrolyte,

- Figure 11 shows the voltammogram recorded under air at pH 11.45 of the same complex, where can be evaluated the oxidation potentials of the dioxygenated complex $((cyclam)Co(OH))_2O_2^{2+}$ and of the $OH^-$ ion, and the reduction potentials of the dissolved dioxygen and of the $(cyclam)Co(OH)^{2+}$ species.
- Figure 12 shows the voltammograms recorded under air, at several pH values for the $(cyclam)Co(OH)^+$ complex, which permit the determination of the optimum pH range for the oxidation of the dioxygenated complex, that is, for this complex, between 11 and 11.9 (curves b,c and d).
- Figure 13 shows the voltammogram recorded under air, at pH 11.9, of the $(cyclam)Co(OH)^+$ complex in a buffered medium containing sulfate ions, and illustrates the influence of the bulk composition on the oxidation and reduction potentials.
- Figure 14, illustrates the electrochemical $O_2$ desorption efficiency as a function of time for the dioxygenated complex $((cyclam)Co(OH))_2O_2^{2+}$ in the presence of sodium perchlorate.

## Claims

1) Process for the separation of a gas from a gas mixture, this mixture being eventually dissolved in a liquid, characterized by the following steps:
- contacting the gas mixture containing the gas to be extracted with a solution, in water or in a solvent mixture that dissolves $OH^-$ ions or an organic base, of a complex LM resulting from the coordination of a metal M, preferably chosen among the following metals: Ni, Co, Cu, Fe, Mn, Cr, V, Zn, and a ligand L of formula

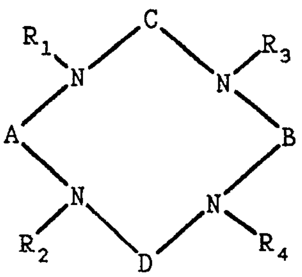

(I)

where,
. the A, B, C and D moieties, identical or different, represent :
- an alkyl chain $-(CH_2)x-$, in which x represents an integer ranging from 2 to 4, substituted or not by one or several groups P or by one or several groups $P_1$, where $P_1$ is an alkyl group of 1 to 4 carbon atoms, substituted or not by one or several groups P, where P represents :
* a -COR group in which R represents a hydroxyle (-OH), a primary amine or an amine substituted by one or two alkyl groups of 1 to 4 carbon atoms, an -OR′ function in which R′ represents an alkyl group of 1 to 4 carbon atoms, or an aromatic cycle of 6 to 14 carbon atoms,
* an aromatic cycle of 6 to 14 carbon atoms, substituted or not in its ortho and/or meta and/or para position by a halide, an alkyl group of 1 to 4 carbon atoms, an alkoxy group of 1 to 4 carbon atoms, a hydroxy, an aryl group, an aromatic heterocycle, a nitro group, a group $-(CH_2)_u-COR$ in which R has the same meaning as above and u is an integer ranging from 0 to 4, or an amine, primary or substituted by 1 to 4 carbon atoms,
* an aromatic heterocycle, especially containing a nitrogen atom, of 4 to 12 carbon atoms, substituted or not in its ortho and/or meta and/or para position by a halide, an alkyl group of 1 to 4 carbon atoms, an alkoxy of 1 to 4 carbon atoms, a hydroxy, an aromatic heterocycle, an aryle group, a nitro group, a group $-(CH_2)_u-COR$ in which R has the meaning indicated above and u represents an integer ranging from 0 to 4, or a primary amine or an amine substituted by one or two alkyl groups of 1 to 4 carbon atoms,
* a primary amine group or an amine substituted by one or two alkyl groups of 1 to 4 carbon atoms,
* a CN group,
* an alkyl group of 1 to 4 carbon atoms,
* an alkoxy group of 1 to 4 carbon atoms,
* a hydroxyl function,
* a nitro group,
* a halide,
- an aromatic cycle of 6 to 14 carbon atoms, in which two carbon atoms respectively participate to a bond

with the nitrogen atoms located on each side of the A,B,C or D moieties of formula (I), this aromatic cycle being substituted or not by one or several groups P and/or $P_1$, where P and $P_1$ have the same meaning as above,

- a group $-CH_2-E-CH_2-$ where E represents an aromatic cycle of 6 to 14 carbon atoms, substituted or not by one or several groups P and/or $P_1$, where P and $P_1$ have the same meaning as above,

. $R_1$, $R_2$, $R_3$ and $R_4$, identical or different, represent a hydrogen atom or a group P or $P_1$, where P and $P_1$ have the same meaning as above,

this contacting being effected during a sufficient length of time for a complete formation of the complexes between the metal complexes LM as described above and the gas molecule to be extracted,

- transferring the obtained bulk in the anode compartment of an electrochemical cell, this cell containing electrodes, a cathode and an anode, respectively of high hydrogen overpotential and of high oxygen overpotential, preferably made of carbon,

- application, between the two cell compartments, of a potential high enough to induce the oxidation of the complexes formed with LM and the gas molecule, the unloading of the gas molecule in the vicinity of the anode and the reduction at the cathode of the complex LM after the above mentioned oxidation,

- recovery of the gas at the exit of the anode compartment, especially with a gas-liquid separation tower,

- if necessary, recovery and recycle of the LM complexes coming out of the cathode compartment into the operating cycle described above.

2) Process according to claim 1, where the pH of the medium containing the complexes of LM with the gas molecule to be separated is adjusted at a value ranging from 7 to 14, and especially from 8 to 12 by addition of a strong inorganic base or by addition of an organic base as long as it does not permit the oxidation of the $OH^-$ ions or of the organic base in the next steps, by adding a strong inorganic base, such as but not limited to sodium or potassium hydroxide or by adding an organic base such as but not limited to 3,5-lutidine, 2,6-lutidine or triethylamine, then by adding an appropriate amount of a buffered solution in the case the added base is an inorganic base.

3) Process according to claim 1 or claim 2, where the following steps are performed before the steps described in claim 1 :

- dissolving ligand L in water or in a solvent mixture where $OH^-$ ions are soluble,

- adding a strong base, especially sodium or potassium hydroxide or an organic non-coordinating base such as but not limited to 2,6-lutidine or 2,4,6-collidine in such a quantity that the pH of the above mentioned solution becomes higher than the value of the $pK_{An}$, i.e. the $(-\log K_{An})$ value where $K_{An}$ represents the last protonation equilibrium constant of ligand L, which is expressed as the ratio of the product of the L and $H^+$ concentrations over the concentration of the monoprotonated ligand $LH^+$, in this solution,

- adding a known amount of a metal salt of formula $M_nX_m$, where M represents a transition metal at the oxidation state m+, and X represents an anion of charge n-, preferably chosen among $Cl^-$,$ClO_4^-$, $NO_3^-$, $CO_3^{2-}$, $SO_4^{2-}$, this amount being greater or preferably equal to one equivalent per ligand equivalent, and leaving the reaction proceed during a sufficient length of time so that the metal complex, LM, is formed

- if necessary, adjusting the pH by adding a strong inorganic base, especially sodium or potassium hydroxide or an organic base, either coordinating or non-coordinating, especially 3,5-lutidine, 2,6-lutidine, 2,4,6-collidine or triethylamine, in order to maintain the pH at the value obtained in the preceeding step.

4) Process according to claim 1 or claim 2, where the solution used in the different steps described above, is bufferd by carbonate or hydrogenocarbonate ions, especially potassium carbonate or hydrogenocarbonate, which can be mixed or not with sulfate ions, especially potassium sulfate.

5) Process according to claim 1 or 2 where the medium containing LM is added of organic bases, and more specifically nitrogeneous organic bases coordinating or non-coordinating or mixtures of coordinating and non-coordinating bases, these bases acting as buffering agents and for the case of a tetracoordinating ligand a coordinating base further acting as an axial ligand.

6) Process according to one of claims 1 to 5 where the potential applied between the two cell compartments is ranging from 0.2 to 2V and more specifically from 0.5 to 1.8V, this potential being slightly higher than the sum of the redox potential value of the species oxidized at the anode, i.e. the complex formed with LM and the gas molecule, and of the redox potential value of the species reduced at the anode, i.e. the LM complex resulting from the electrochemical desorption.

7) Process according to one of claims 1 to 6 where the gas to extract from a gas mixture is the gas to produce and is collected at the exit of the anode compartment.

8) Process according to one of claims 1 to 7, performed under a pressure ranging from 1 to 200 bar and preferably ranging from 2 to 20 bar, in such a way that the expected gas is collected and stored under pressure.

9) Process according to one of claims 1 to 8 where the expected produced gas is the dioxygen from air and

the metal M complexed to the ligand L is the cobalt.

10) Process according to one of claims 1 to 8 where the expected produced gas is the carbon dioxide from a gas mixture and the metal M complexed to ligand L is chosen among metals such as Co, Cu, Ni, Zn, Ru, Pt and preferably among metals such as Cu and Zn.

11) Process according to one of claims 1 to 8 where the expected produced gas is the carbon monoxide from a gas mixture and the metal M complexed to the ligand L is chosen among metals such as Cu, Ni, Rh, Ir and preferably M is Cu.

12) Process according to one of claims 1 to 8, where the expected produced gas is the nitrogen monoxide from a gas mixture and the metal M complexed to the ligand L is chosen among metals such as Mn, Fe, Co and preferably M is Co.

13) Process according to one of claims 1 to 8 where the expected produced gas is the hydrogen from a gas mixture and the metal M complexed to the ligand L is chosen among the metals Pd, Pt, Ru, Mo and preferably M is Mo.

14) Process according to one of claims 1 to 6 where the gas extracted from a gas mixture is an impurity that must be eliminated, such a process being applied to the purification of a gas or of a gas mixture by elimination of the extracted gas at the exit of the anode compartment and by collecting the purified gas or mixture of gases at the exit of the absorption tower before the anode compartment.

15) Process according to claim 14, performed under a pressure ranging from 1 to 200 bar and preferably from 2 to 20 bar, where the purified gas or mixture of gases is collected and stored under pressure.

Neu eingereicht / Newly filed
Nouvellement déposé

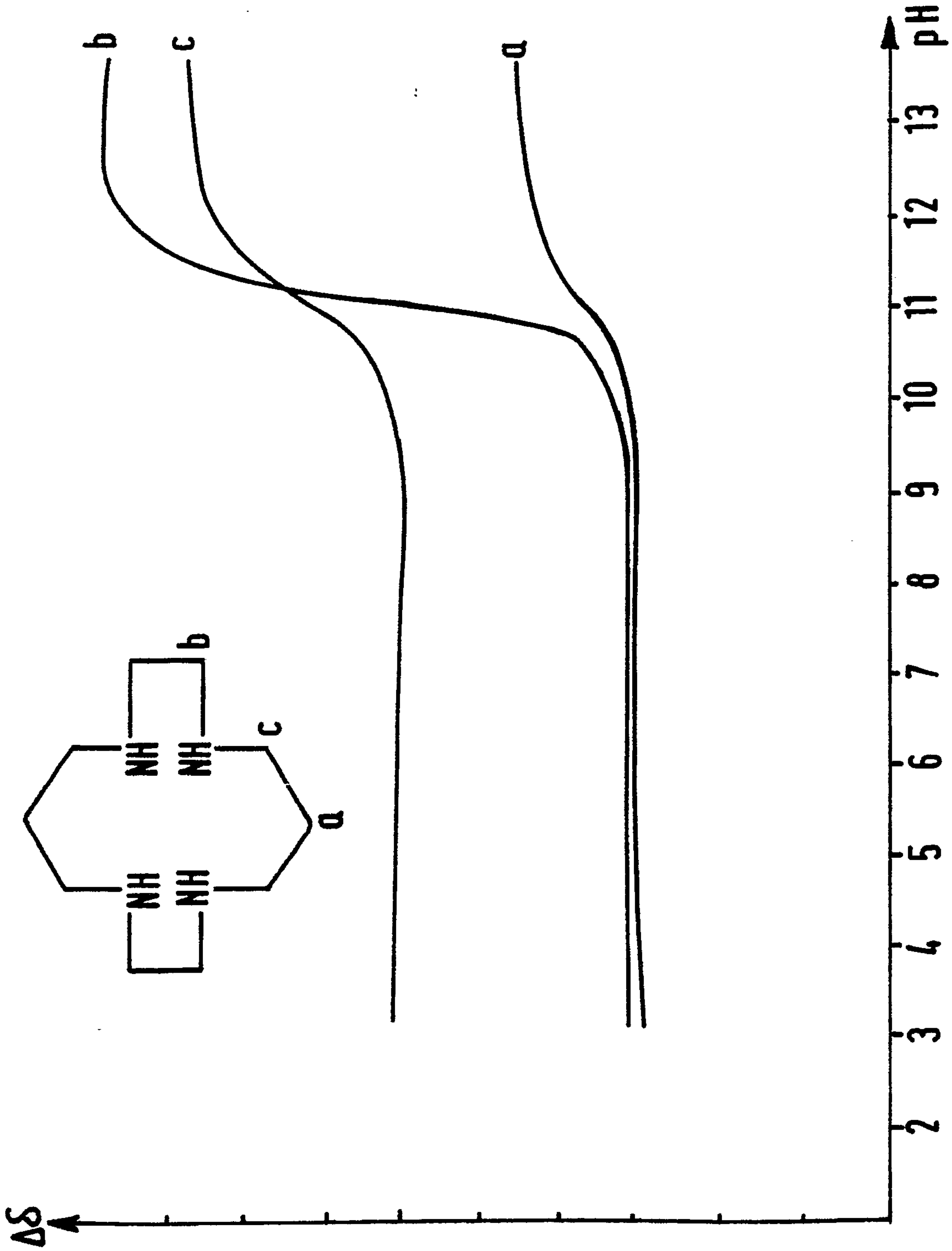


**FIG.1: TITRATION CURVE, $\Delta S_{HI} = f(pH)$ (ligand 1)**

Neu eingereicht / New...
Nouvellement déposé

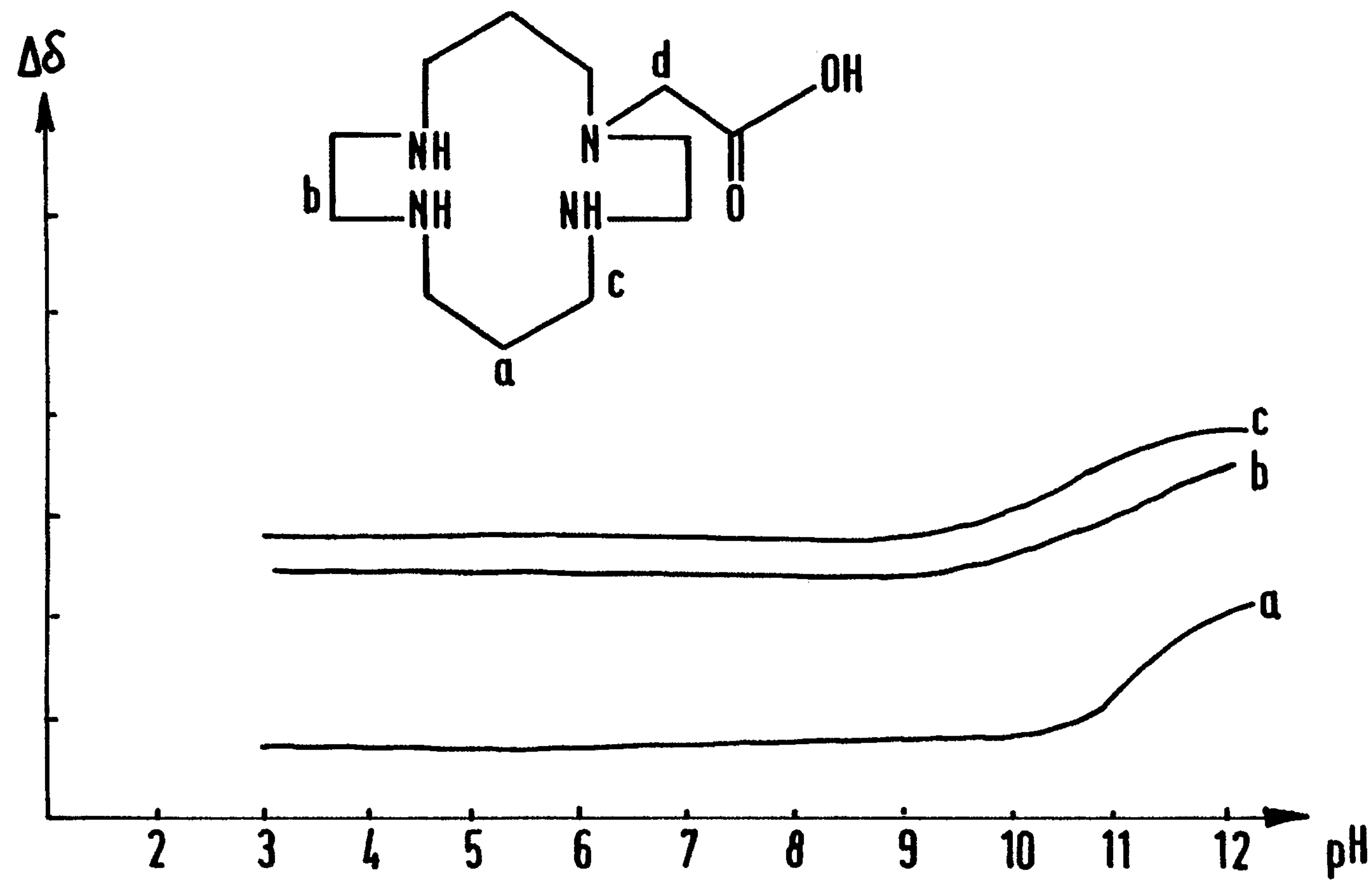


FIG.2 : TITRATION CURVE $\Delta\delta_{Hi} = f(pH)$ (ligand 2)

Neu eingereicht / Newly filed
Nouvellement déposé

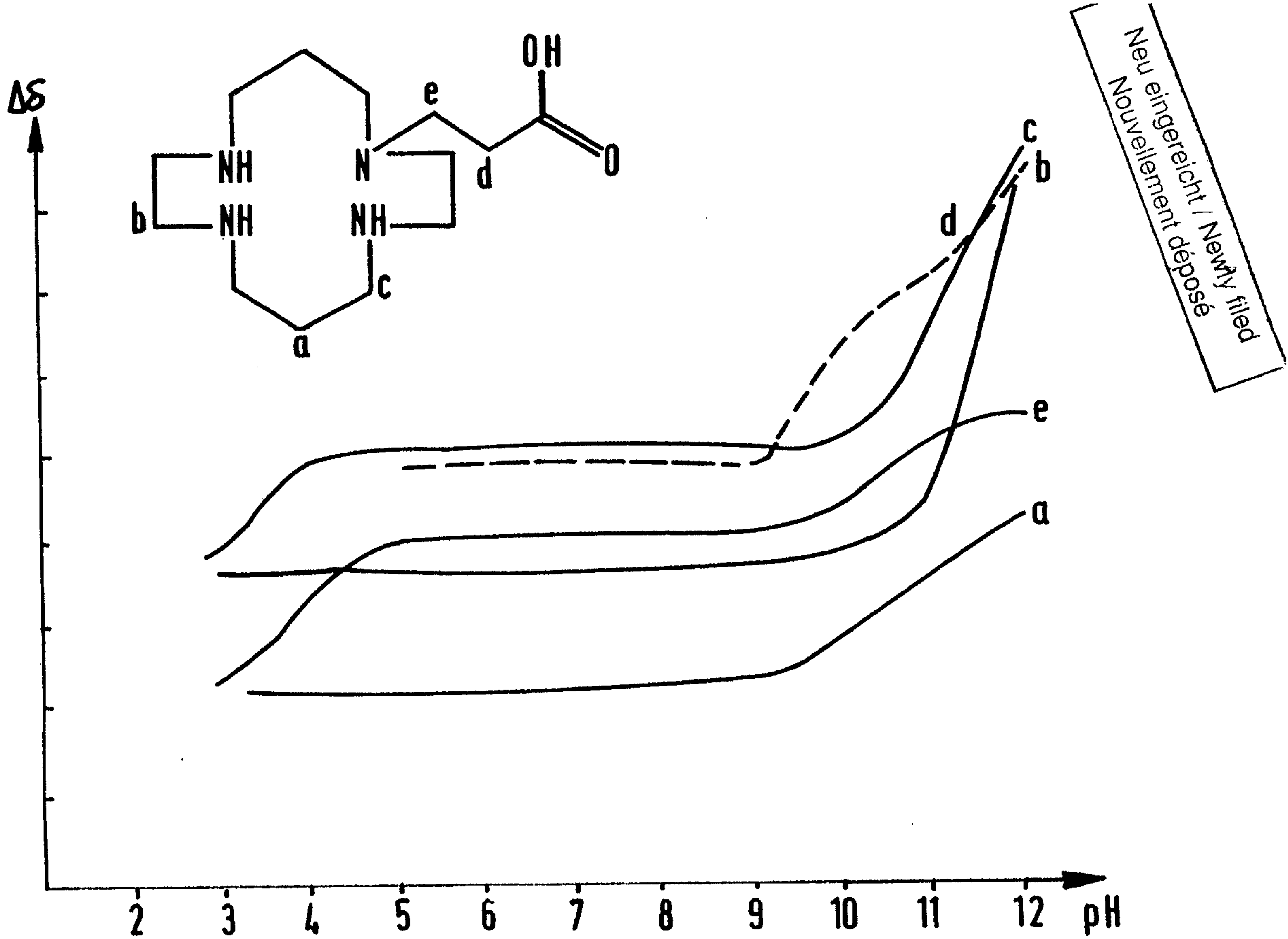


FIG.3: TITRATION CURVE $\Delta\delta_{Hi} = f(pH)$ (ligand 3)

Neu eingereicht / Newly filed
Nouvellement déposé

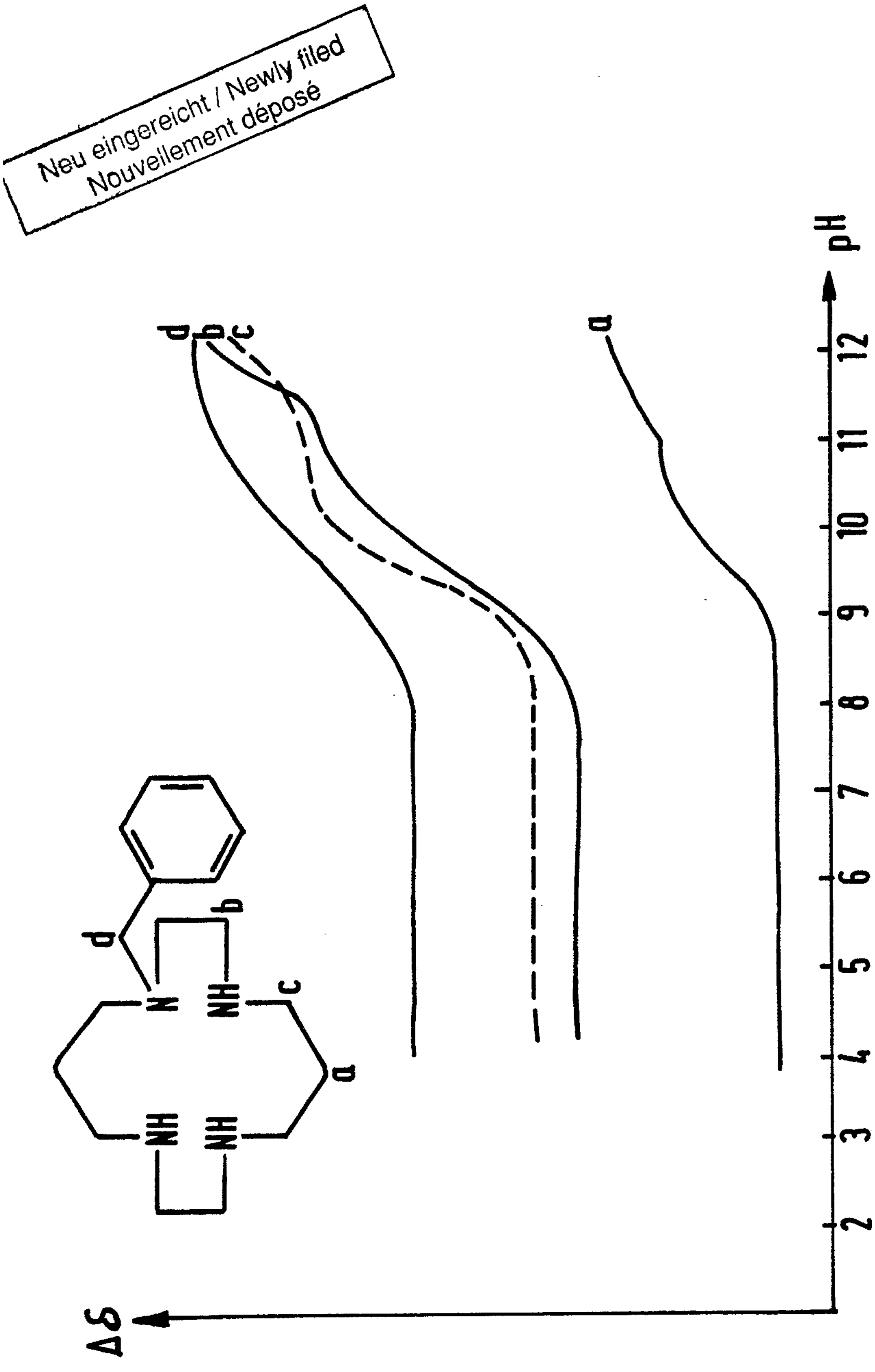


**FIG. 4**: Titration Curve $\Delta\delta_{Hi} = f(pH)$ (ligand 4)

Neu eingereicht / Newly filed
Nouvellement déposé

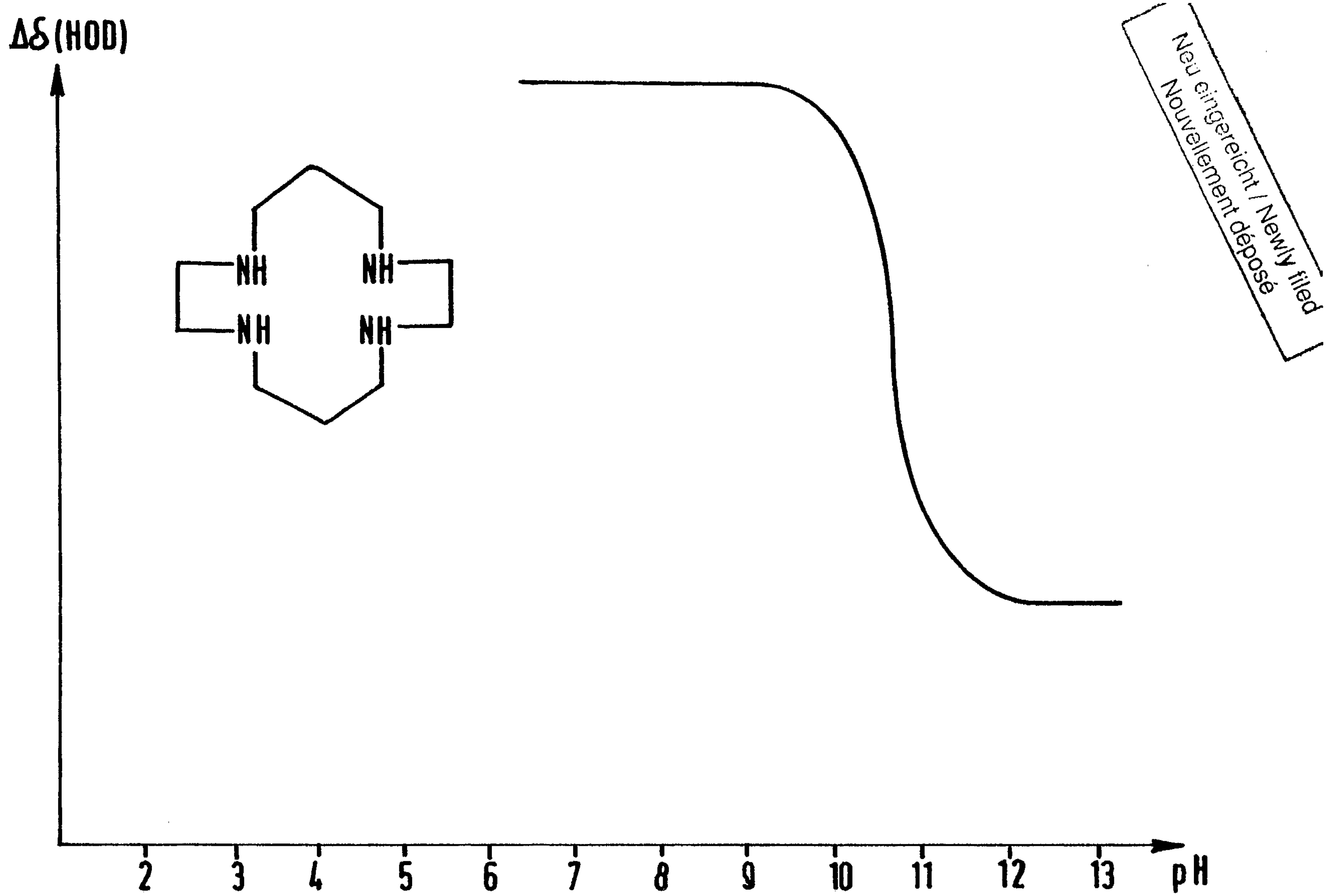


**FIG. 5:** Titration Curve $\Delta\delta_{HOD} = f(pH)$ (ligand 1)

Neu eingereicht / Newly filed
Nouvellement déposé

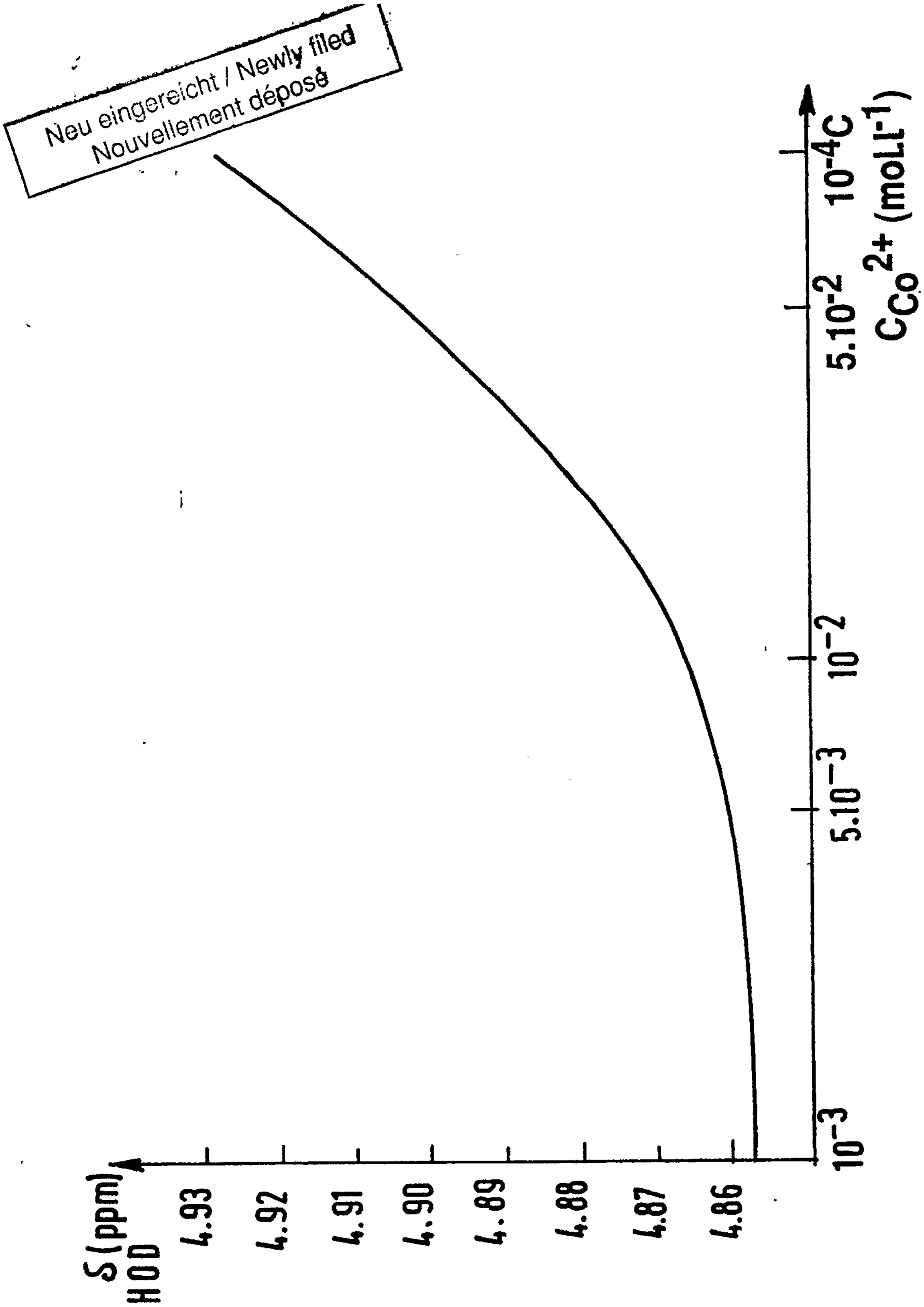


**FIG.6 : STANDARD CURVE $\Delta S_{HOD}$ = f [contration Co(2+)]**

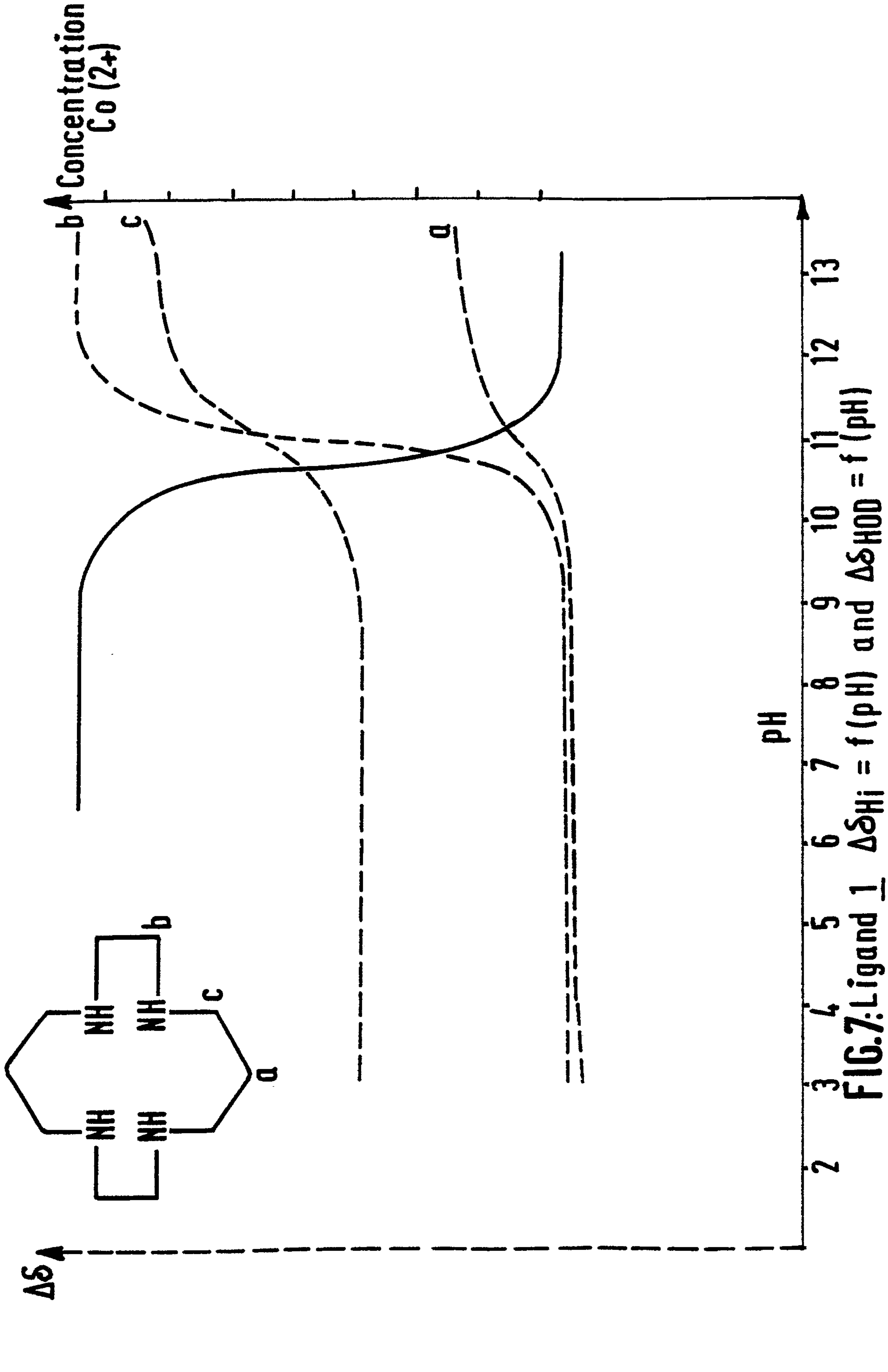


FIG.7: Ligand 1 $\Delta\delta_{Hi} = f(pH)$ and $\Delta\delta_{HOD} = f(pH)$

Neu eingereicht / Newly filed
Nouvellement déposé

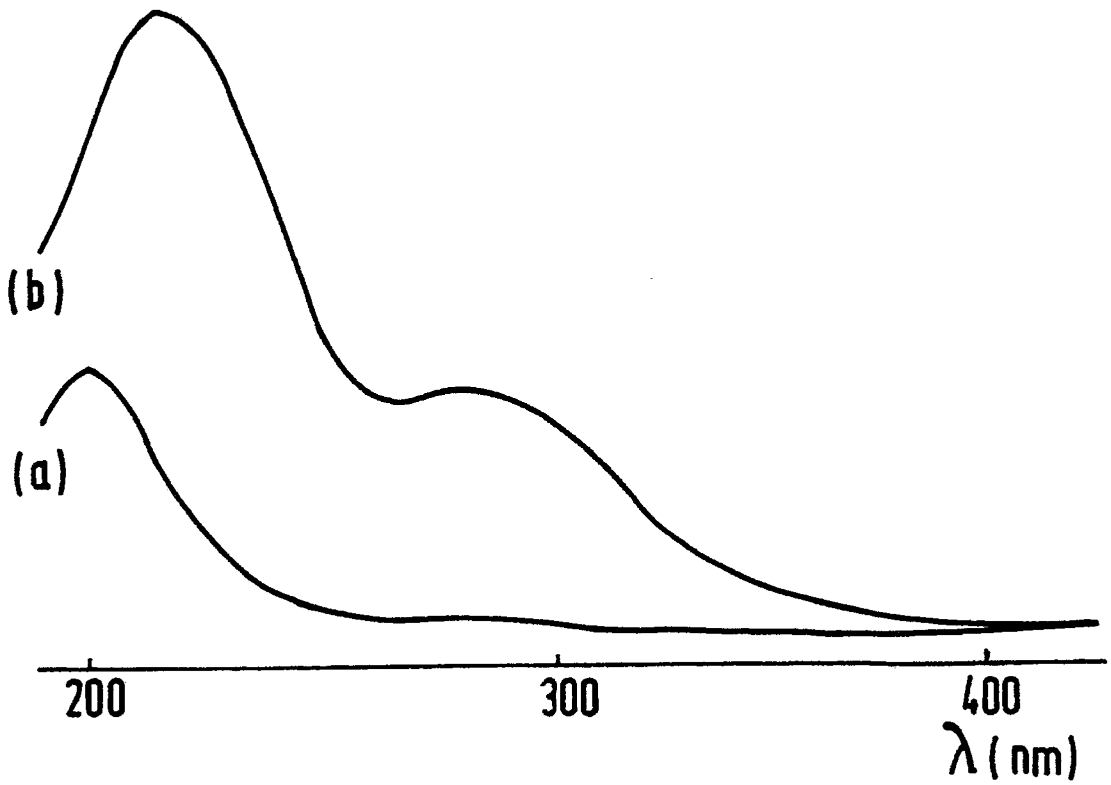


FIG.8: UV-Visible spectra of complex $LCo^{2+}$ (L = 1) under argon atmosphere (a) and under oxygen (b).

eu eingereicht / Nouvellement déposé

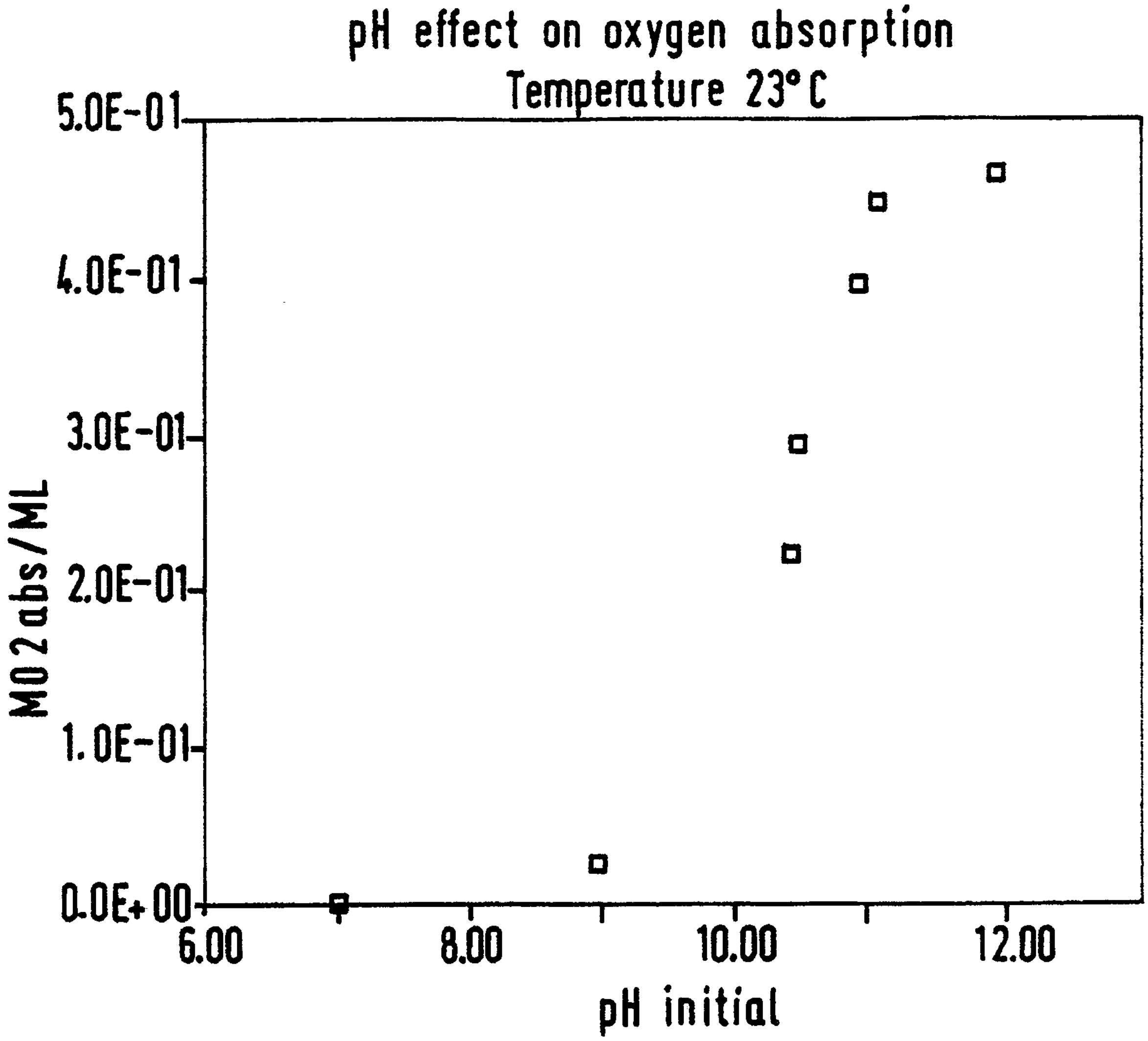


**FIG.9:** Variation of the $MO_2abs/M_L$ ration as a function of pH ( L = 1).

Neu eingereicht / Newly filed
Nouvellement déposé

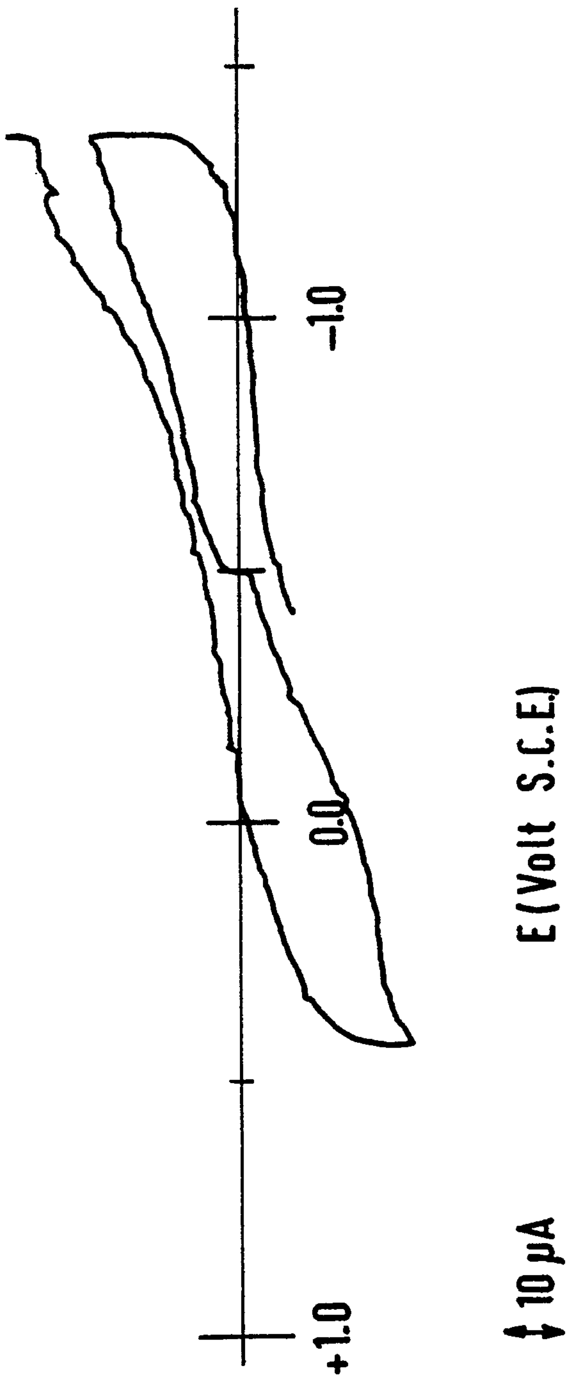


10mM

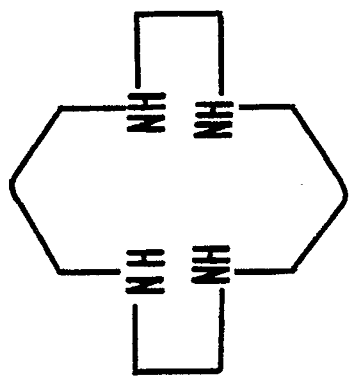

+10mM $Co^{II}(ClO_4)_2$
+0.5M $NaClO_4$

FIG.10

Neu eingereicht / Newly filed
Nouvellement déposé

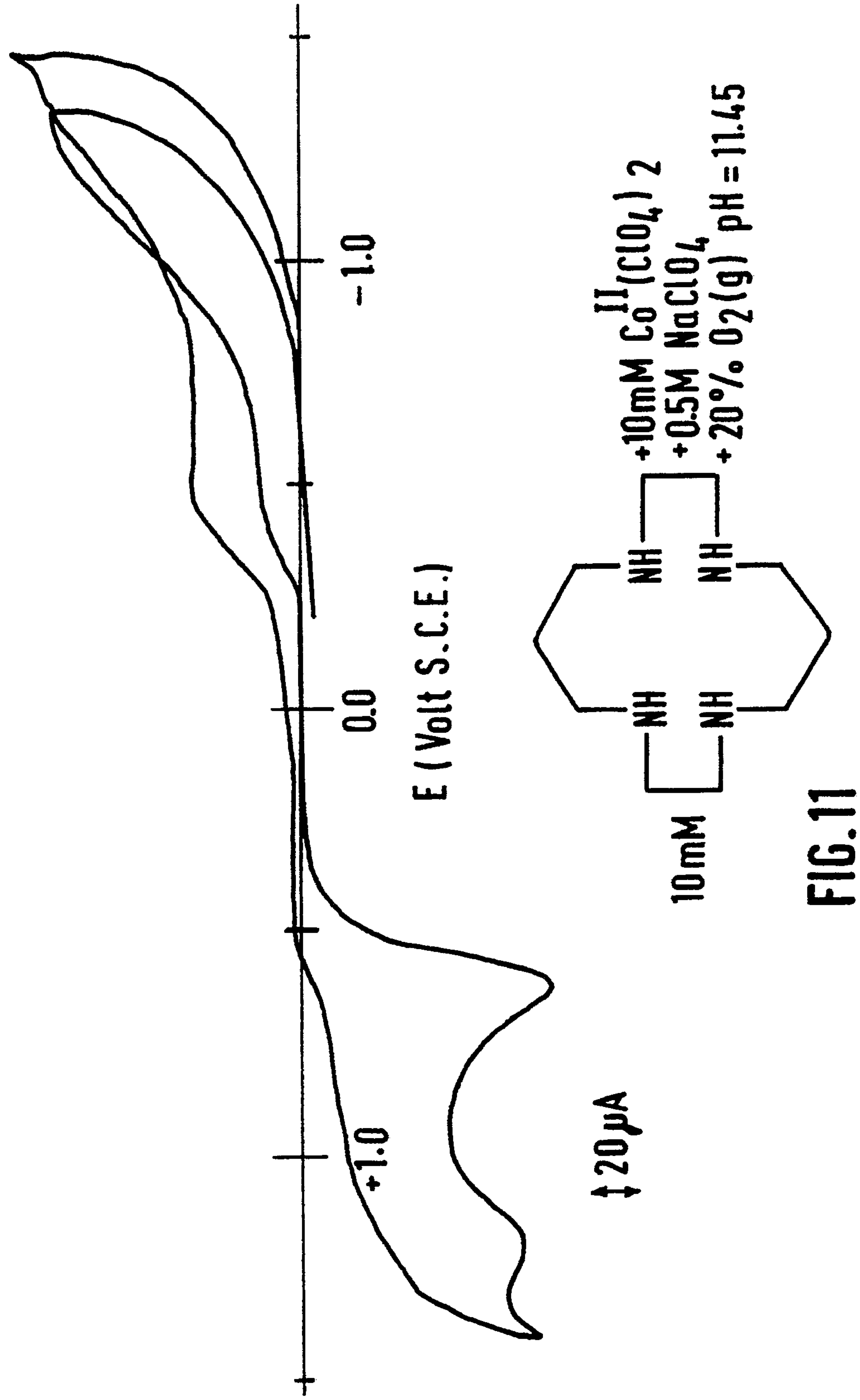


FIG.11

Neu eingereicht / Newly filed
Nouvellement déposé

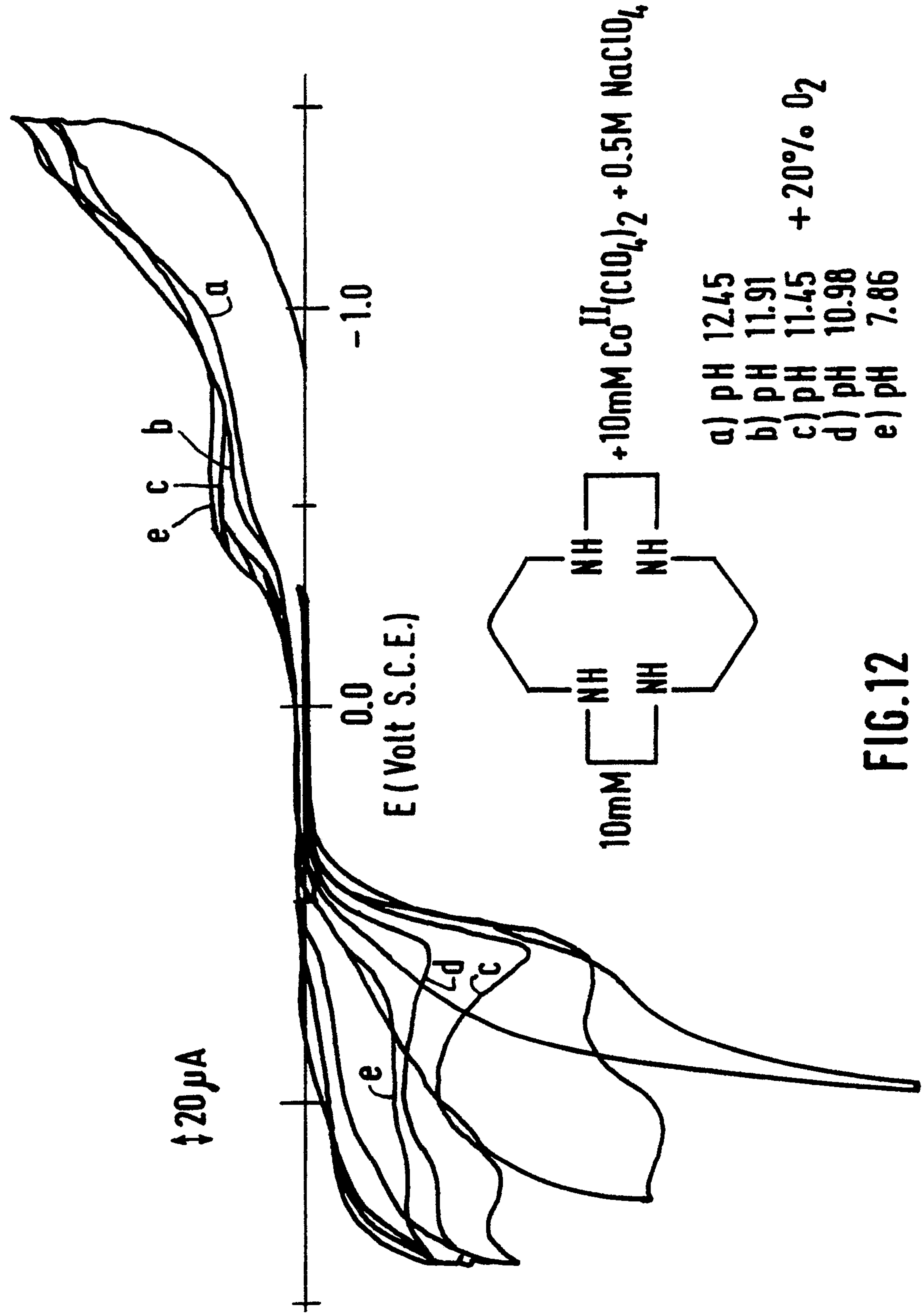


FIG.12

Neu eingereicht / Newly filed
Nouvellement déposé

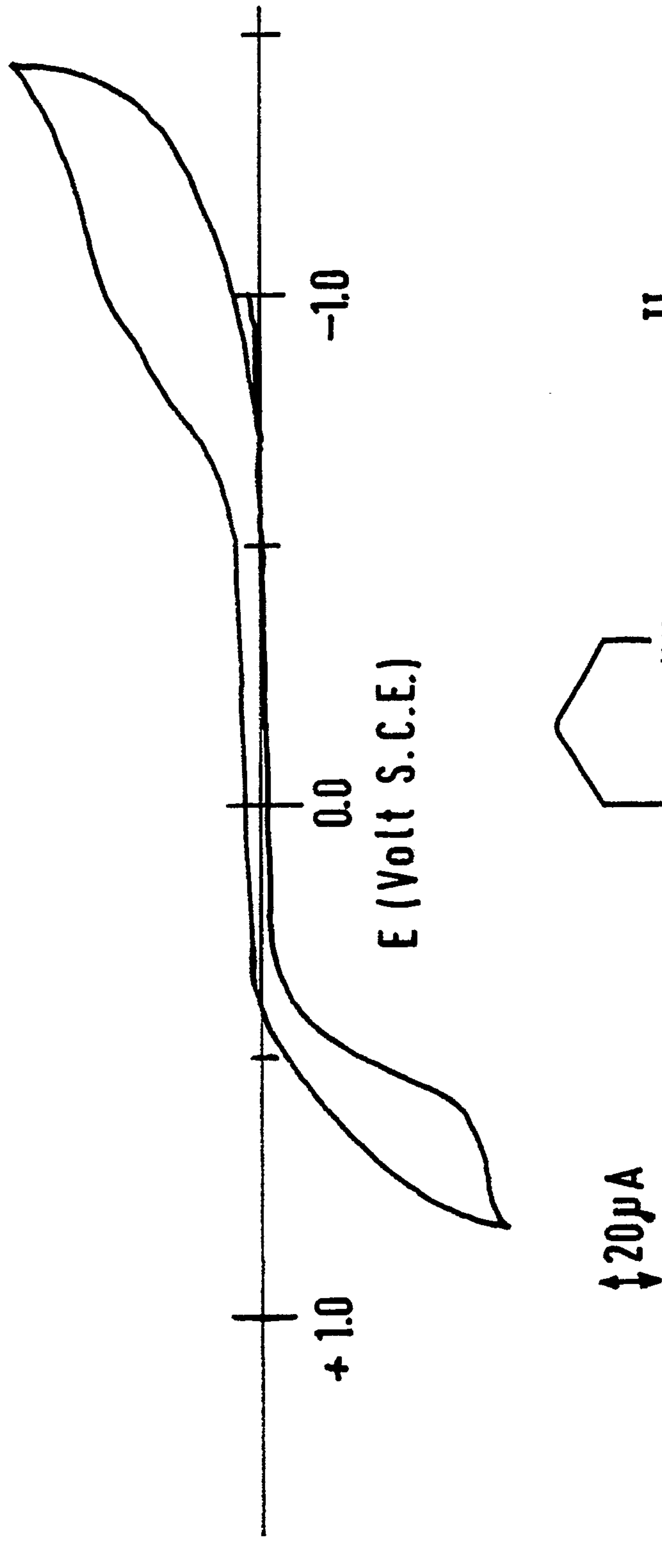


10mM

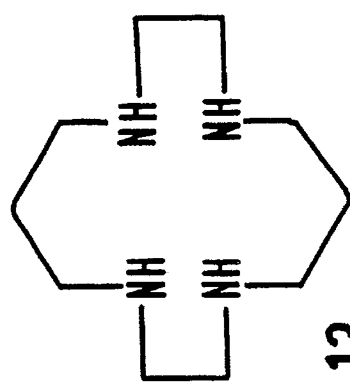

+10mM $Co^{II}(SO_4)$
+0.25M $K_2SO_4$+0.25M $K_2CO_3$
+20% $O_2$(g) pH 11.90

FIG.13

Neu eingereicht / Newly filed
Nouvellement déposé

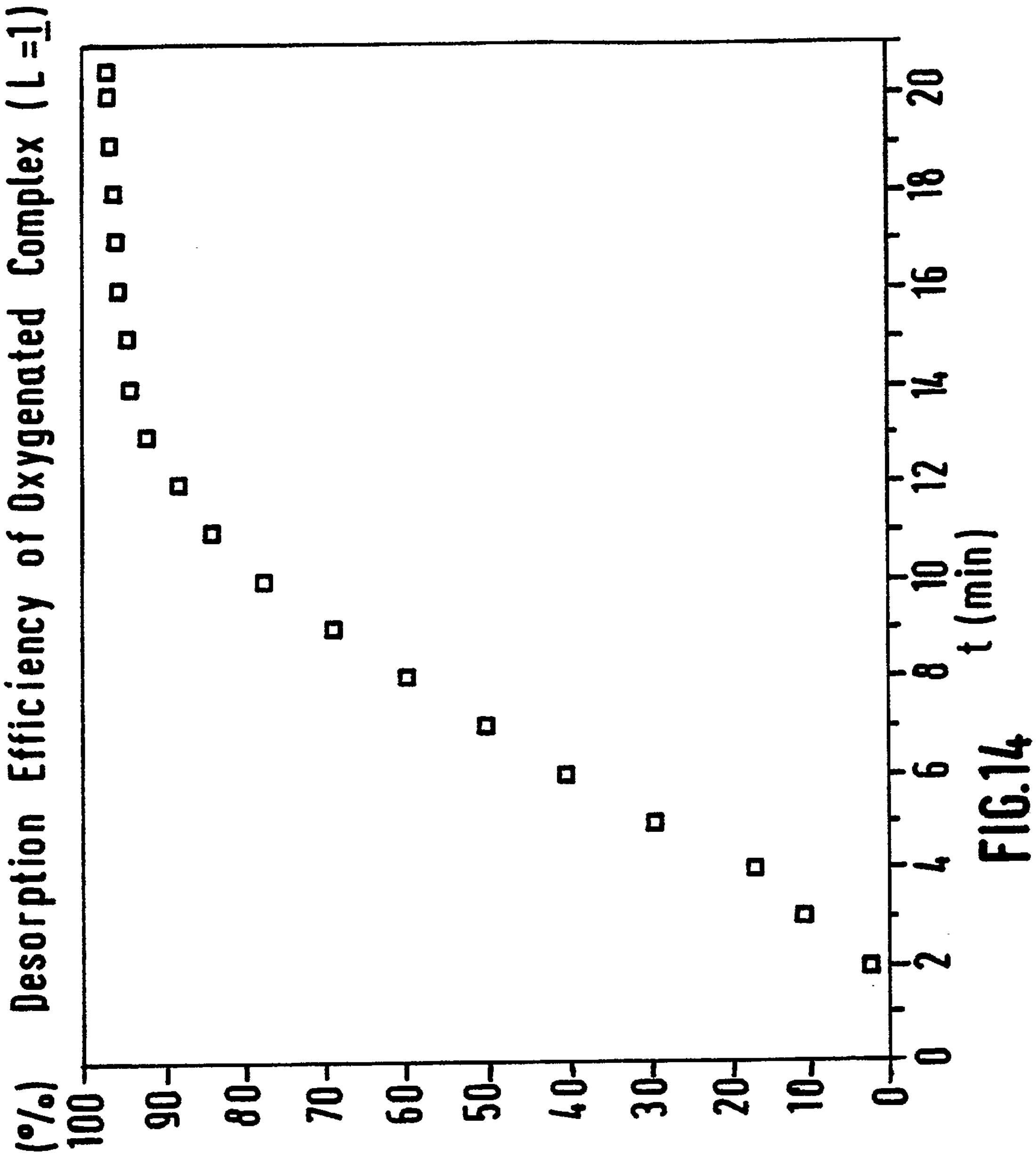


FIG.14

European Patent Office

# EUROPEAN SEARCH REPORT

Application Number

EP 90 40 3149

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A,D | JOURNAL OF THE AMERICAN CHEMICAL SOCIETY vol. 103, no. 24, 2 December 1981, EASTON, USA pages 7489 - 7496; T.GEIGER et al.: "Homogeneous Catalysis of the Electrochemical Reduction of Dioxygen by a Macrocyclic Cobalt III Complex" * the whole document * --- | 1, 3, 6, 9, 14 | B01D53/14<br>C01B13/02 |
| A,D | EP-A-283751 (AQUANAUTICS CORP.) * claims 1-29 * --- | 1-7, 9, 10 | |
| A | US-A-4680037 (D.RAMPRASAD ET AL.) * claims 1-3, 15 * ----- | 1 | |
| | | | TECHNICAL FIELDS SEARCHED (Int. Cl.5 ) |
| | | | B01D<br>C01B |

The present search report has been drawn up for all claims

| Place of search | Date of completion of the search | Examiner |
|---|---|---|
| BERLIN | 14 DECEMBER 1990 | BERTRAM H.E.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document



EPO FORM 1503 03.82 (P0401)